(19) Europäisches Patentamt
European Patent Office
Office européen des brevets

(11) EP 0 552 766 B1

(12) EUROPÄISCHE PATENTSCHRIFT

(45) Veröffentlichungstag und Bekanntmachung des Hinweises auf die Patenterteilung:
**22.05.2002 Patentblatt 2002/21**

(51) Int Cl.[7]: **C07H 21/00**, C07F 9/24, A61K 31/70, C12Q 1/68

(21) Anmeldenummer: **93100892.4**

(22) Anmeldetag: **21.01.1993**

(54) **Oligonucleotidanaloga, deren Herstellung und Verwendung**

Oligonucleotide analogues, their preparation and use

Analogues oligonucléotidiques, leur préparation et leur utilisation

(84) Benannte Vertragsstaaten:
**AT BE CH DE DK ES FR GB GR IE IT LI LU MC NL PT SE**

(30) Priorität: **22.01.1992 DE 4201662**

(43) Veröffentlichungstag der Anmeldung:
**28.07.1993 Patentblatt 1993/30**

(73) Patentinhaber: **Aventis Pharma Deutschland GmbH**
**65929 Frankfurt am Main (DE)**

(72) Erfinder:
- **Uhlmann, Eugen, Dr.**
  **W-6246 Glashütten (DE)**
- **Peyman, Anuschirwan, Dr.**
  **W-6233 Kelkheim7Ts. (DE)**
- **O'Malley, Gerard, Dr.**
  **Newtown, Pennsylvania 18940 (US)**
- **Helsberg, Matthias, Dr.**
  **W-6233 Kelkheim/Ts (DE)**
- **Winkler, Irvin, Dr.**
  **W-6237 Liederbach (DE)**

(56) Entgegenhaltungen:
**EP-A- 0 289 619** **EP-A- 0 294 196**
**WO-A-84/01778** **WO-A-86/07361**
**WO-A-91/06556** **WO-A-92/02638**
**WO-A-92/06103**

- **GENE, Bd.61, 1987 Seiten 307 - 315 P.VERSPIEREN ET AL. 'An Acridine-linked Oligodeoxynucleotide Targeted to the Common 5'-end of Trypanosome mRNAs Kills Cultured Parasites.'**
- **BIOCONJUGATE CHEMISTRY, Bd.1, Nr.3, 1990 Seiten 165 - 187 J.GOODCHILD 'Conjugates of Oligonucleotides and Modified Oligonucleotides: A Review of Their Synthesis and Properties.'**
- **CHEMICAL REVIEWS, Bd.90, Nr.4, 1990, WASHINGTON Seiten 543 - 584 E.UHLMANN ET AL. 'Antisense Oligonucleotides: A New Therapeutic Principle.'**
- **BULLETIN DE LA SOCIETE CHIMIQUE DE FRANCE. 2 PARTIE - CHIMIE ORGANIQUE, BIOCHIMIE, Nr.1-2, 1981, PARIS FR Seiten II-51 - II-56 N.T.THUONG ET AL. 'Nouvelle Méthode de préparation d'esters Phosphoriques, Renfermant un Groupe B-Mercaptoéthyle, Utilisables en Synthèse Nucléotidique.'**

Bemerkungen:
Die Akte enthält technische Angaben, die nach dem Eingang der Anmeldung eingereicht wurden und die nicht in dieser Patentschrift enthalten sind.

EP 0 552 766 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft neue Oligonucleotidanaloga mit wertvollen physikalischen, biologischen und pharmakologischen Eigenschaften sowie ein Verfahren zu deren Herstellung. Ihre Anwendung bezieht sich auf die Verwendung als Inhibitoren der Genexpression (Antisense Oligonucleotide, Ribozyme, Sense Oligonucleotide und Triplex Forming Oligonucleotide), als Sonden zum Nachweis von Nucleinsäuren und als Hilfsmittel in der Molekularbiologie.
Oligonucleotide finden in wachsendem Maße Anwendung als Inhibitoren der Genexpression (G. Zon, Pharmaceutical Research 5, 539 (1988); J. S. Cohen, Topics in Molecular and Structural Biology 12 (1989) Macmillan Press; C. Helene and J. J. Toulme, Biochemica et Biophysica Acta 1049, 99 (1990); E. Uhlmann and A. Peyman, Chemical Reviews 90, 543 (1990)). Antisense Oligonucleotide sind Nucleinsäure-Fragmente, deren Basensequenz komplementär ist zu einer zu inhibierenden mRNA. Diese Target-mRNA kann zellulären, viralen oder sonstigen pathogenen Ursprungs sein. Als zelluläre Target-Sequenzen kommen beispielsweise die von Rezeptoren, Enzymen, Immunmodulatoren, Ionenkanälen oder Onkogenen in Frage. Die Inhibition der Virus Vermehrung mit Hilfe von Antisense Oligonucleotiden wurde beispielsweise für RSV (Rous Sarcoma Virus), HSV-1 und -2 (Herpes Simplex Virus Typ I und II), HIV (Human Immunodeficiency Virus) und Influenza-Viren beschrieben. Dabei setzt man Oligonucleotide ein, die zur viralen Nucleinsäure komplementär sind. Sense Oligonucleotide sind dagegen in ihrer Sequenz so konzipiert, daß sie beispielsweise Nucleinsäure-bindende Proteine oder Nucleinsäure-prozessierende Enzyme binden ("einfangen") und so deren biologische Aktivität inhibieren (Helene, 1990). Als virale Targets sind hier beispielsweise die Reverse Transkriptase, DNA-Polymerase und Transaktivator-Proteine zu nennen. Triplex Forming Oligonucleotide haben im allgemeinen die DNA als Target und bilden nach Bindung an diese eine tripelhelicale Struktur aus. Während mit Hilfe der Antisense Oligonucleotide im allgemeinen die Prozessierung (Splicing etc.) der mRNA oder deren Translation in das Protein gehemmt werden, hemmen Triplex Forming Oligonucleotide die Transkription oder Replikation der DNA (Helene et al., 1990, Uhlmann und Peyman, 1990). Es ist aber auch möglich, einzelsträngige Nucleinsäuren in einer ersten Hybridisierung mit einem Antisense Oligonucleotid unter Ausbildung eines Doppelstranges zu binden, der dann in einer zweiten Hybridisierung mit einem Triplex Forming Oligonucleotid eine Triplex-Struktur ausbildet. Die Antisense und Triplex Bindungsregionen können dabei entweder in zwei separaten Oligonucleotiden oder aber in einem Oligonucleotid beherbergt sein. Eine weitere Anwendung synthetischer Oligonucleotide sind die sogenannten Ribozyme, welche die Target-RNA infolge ihrer Ribonuclease-Aktivität zerstören (J.J. Rossi and N. Sarver, TIBTECH 8, 179 (1990).
In der DNA-Diagnostik werden Nucleinsäure-Fragmente mit geeigneter Markierung als sogenannte DNA-Sonden oder DNA-Probes für die spezifische Hybridisierung an eine nachzuweisende Nucleinsäure eingesetzt. Die spezifische Ausbildung des neuen Doppelstranges wird dabei mit Hilfe der Markierung, die vorzugsweise nicht radioaktiv ist, verfolgt. Auf diese Weise lassen sich genetische, maligne, virale oder durch andere Pathogene verursachte Krankheiten nachweisen.

Für die meisten genannten Anwendungen sind Oligonucleotide in ihrer natürlich vorkommenden Form wenig oder völlig ungeeignet. Sie müssen chemisch so modifiziert werden, daß sie den speziellen Anforderungen gerecht werden. Damit Oligonucleotide in biologischen Systemen, beispielsweise zur Inhibition der Virus-Vermehrung eingesetzt werden können, müssen sie folgende Voraussetzungen erfüllen:

1. Sie müssen unter in vivo Bedingungen, also sowohl im Serum als auch intrazellulär, eine ausreichend große Stabilität aufweisen.
2. Sie müssen so beschaffen sein, das sie die Zell- und Nucleus-Membran passieren können.
3. Sie müssen unter physiologischen Bedingungen in Basen-spezifischer Weise an ihre Target-Nucleinsäure binden, um den inhibitorischen Effekt zu entfalten.

Für DNA-Sonden sind diese Voraussetzungen nicht unabdingbar; jedoch müssen diese Oligonucleotide so derivatisiert sein, daß ein Nachweis, beispielsweise mittels Fluoreszenz, Chemilumineszenz, Kolorimetrie oder spezifischer Färbung, möglich ist (Beck und Köster, Anal. Chem. 62, 2258 (1990).
Die chemische Veränderung der Oligonucleotide erfolgt meistens in der Weise, daß Phosphatrückgrat, Ribose-Einheit oder die Nucleobasen entsprechend verändert 2werden (Cohen, 1989; Uhlmann und Peyman, 1990). Eine weitere häufig genutzte Methode ist die Herstellung von Oligonucleotid-5'-Konjugaten durch Umsetzung der 5'-Hydroxy-Gruppe mit entsprechenden Phosphorylierungs-Reagenzien. Oligonucleotide, die nur am 5'-Ende modifiziert sind, haben den Nachteil, daß sie im Serum abgebaut werden. Wenn dagegen alle Internucleotid-Phosphat-Reste verändert werden, verändern sich die Eigenschaften der Oligonuleotide oft drastisch. Beispielsweise ist die Löslichkeit der Methylphosphonat Oligonucleotide in wäßrigem Medium vermindert und das Hybridisierungsvermögen reduziert. Phosphorothioat-Oligonucleotide wirken unspezifisch, so daß beispielsweise auch Homooligomere gegen Viren wirksam sind.
Aufgabe ist es daher, Oligonucleotidanaloga mit spezifischer Wirksamkeit, erhöhter Serumstabilität und guter Löslichkeit bereitzustellen.

Gegenstand der Erfindung sind Oligonucleotidanaloga der Formel I mit einer modifizierten 3'- bzw. 2'-terminalen Phosphatgruppe

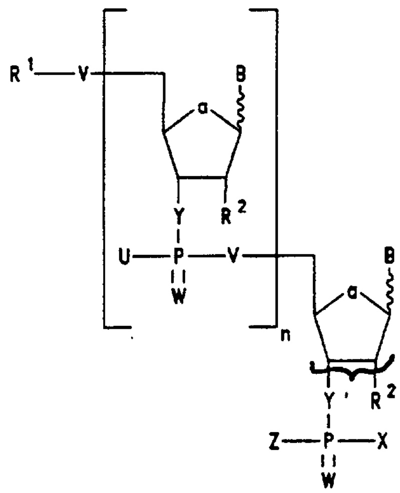

(I)

sowie deren physiologisch verträglichen Salze, worin

$R^1$ Wasserstoff, $C_1$-$C_{18}$-Alkyl, vorzugsweise $C_1$-$C_6$-Alkyl, $C_2$-$C_{18}$-Alkenyl, $C_2$-$C_{18}$-Alkinyl, $C_2$-$C_{18}$-Alkylcarbonyl, $C_3$-$C_{19}$-Alkenylcarbonyl, $C_3$-$C_{19}$-Alkinylcarbonyl, $C_6$-$C_{20}$-Aryl, ($C_6$-$C_{14}$)-Aryl-($C_1$-$C_8$)-alkyl, oder einen Rest der Formel II

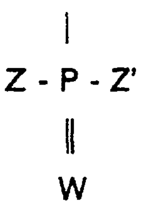

(II)

bedeutet;

$R^2$ Wasserstoff, Hydroxy, $C_1$-$C_{18}$-Alkoxy, Halogen, Azido oder $NH_2$ bedeutet;

B für eine in der Nucleotidchemie übliche Base, beispielsweise für natürliche Basen wie Adenin, Cytosin, Guanin und Thymin oder unnatürliche Basen wie Purin, 2.6-Diaminopurin, 7-Deazaadenin, 7-Deazaguanin, $N^4N^4$-Ethanocytosin, $N^6N^6$-Ethano-2.6-diaminopurin, Pseudoisocytosin, steht;

a für Oxy oder Methylen steht;

n eine ganze Zahl von 1 bis 100, bevorzugt 10 bis 40, bedeutet;

W Oxo, Thioxo oder Selenoxo bedeutet;

V Oxy, Sulfandiyl oder Imino bedeutet;

Y Oxy, Sulfandiyl, Imino oder Methylen bedeutet;

Y' Oxy, Sulfandiyl, Imino, $(CH_2)_m$ oder $V(CH_2)_m$ ist, worin

m eine ganze Zahl von 1 bis 18, vorzugsweise von 1 bis 6, bedeutet;

X Hydroxy oder Mercapto bedeutet;

U Hydroxy, Mercapto, SeH, $C_1$-$C_{18}$-Alkoxy, vorzugsweise $C_1$-$C_6$-Alkoxy, $C_1$-$C_{18}$-Alkyl, vorzugsweise $C_1$-$C_6$-Alkyl, $C_6$-$C_{20}$-Aryl, ($C_6$-$C_{14}$)-Aryl-($C_1$-$C_8$)-alkyl, $NHR^3$, $NR^3R^4$ oder einen Rest der Formel III $(OCH_2CH_2)_pO(CH_2)_qCH_2R^{11}$ (III) bedeutet, worin

$R^3$ $C_1$-$C_{18}$-Alkyl, vorzugsweise $C_1$-$C_8$-Alkyl, $C_6$-$C_{20}$-Aryl, ($C_6$-$C_{14}$)-Aryl-($C_1$-$C_8$)-alkyl, $-(CH_2)_c-[NH(CH_2)_c]_d-NR^{12}R^{12}$, worin c eine ganze Zahl von 2 bis 6 und d eine ganze Zahl von 0 bis 6 ist, und $R^{12}$ unabhängig voneinander Wasserstoff oder $C_1$-$C_6$Alkyl oder $C_1$-$C_4$-Alkoxy-$C_1$-$C_6$-alkyl, bevorzugt Methoxyethyl, ist;

$R^4$ $C_1$-$C_{18}$-Alkyl, vorzugsweise $C_1$-$C_8$-Alkyl und besonders bevorzugt $C_1$-$C_4$-Alkyl, $C_6$-$C_{20}$-Aryl oder ($C_6$-$C_{10}$)-Aryl-($C_1$-$C_8$)-alkyl bedeutet oder im Falle von $NR^3R^4$ zusammen mit $R^3$ und dem sie tragenden Stickstoffatom einen 5-6-gliedrigen heterozyklischen Ring bedeutet, der zusätzlich ein weiteres Heteroatom aus der Reihe O,

S, N enthalten kann,

p eine ganze Zahl von 1 bis 100, bevorzugt 3 bis 20 und besonders bevorzugt 3 bis 8, ist,

q eine ganze Zahl von 0 bis 22, bevorzugt 0 bis 15, ist,

$R^{11}$ Wasserstoff oder eine funktionelle Gruppe wie Hydroxy, Amino, $NHR^{13}$, COOH, $CONH_2$, $COOR^{12}$ oder Halogen bedeutet, worin $R^{12}$ $C_1$-$C_4$-Alkyl, vorzugsweise Methyl, bedeutet;

Z = Z'

Mercapto, $C_1$-$C_{22}$-Alkoxy, vorzugsweise $C_6$-$C_{18}$-Alkoxy, -O-$(CH_2)_b$-$NR^{12}R^{13}$, worin b eine ganze Zahl von 1 bis 6 ist, und $R^{13}$ $C_1$-$C_6$-Alkyl ist oder $R^{12}$ und $R^{13}$ zusammen mit dem sie tragenden Stickstoffatom einen 3-6 gliedrigen Ring bilden, $C_1$-$C_{18}$-Alkyl, vorzugsweise $C_1$-$C_8$-Alkyl,

($C_6$-$C_{14}$)-Aryl-($C_1$-$C_8$)-alkoxy, vorzugsweise ($C_6$-$C_{10}$)-Aryl-($C_1$-$C_4$)-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, $C_1$-$C_4$-Alkylamino, $C_1$-$C_6$-Alkoxy, Hydroxy, Halogen und Cyano substituiert ist, oder einen Rest der Formel III (siehe oben, siehe V) bedeutet, und die geschweifte Klammer andeutet, daß sich $R^2$ und der benachbarte Phosphorylrest in 2'- und 3'-Stellung oder auch umgekehrt in 3'- und 2'-Stellung befinden können,

wobei jedes Nucleotid in seiner D- bzw. L-Konfiguration vorliegen kann und sich die Base B in α- bzw. β-Stellung befinden kann, mit der Maßgabe, daß falls Z = Mercapto, $C_1$-$C_{18}$-Alkyl oder Ethoxy ist, mindestens eine der Gruppen X, Y, Y', V, W ungleich Hydroxy, Oxy bzw. Oxo ist oder $R^1$ ungleich Wasserstoff ist (siehe WO-A-8 401 778).

Bevorzugt sind Oligonucleotidanaloga der Formel I sowie deren physiologisch verträglichen Salze, worin sich die Base B in β-Stellung befindet, die Nucleotide in der D-Konfiguration vorliegen, $R^2$ sich in 2'-Stellung befindet und a für Oxy steht.

Besonders bevorzugt sind Oligonucleotidanaloga der Formel I, worin

$R^1$ Wasserstoff, $C_1$-$C_6$-Alkyl, insbesondere Methyl, oder einen Rest der Formel II bedeutet;

$R^2$ Wasserstoff oder Hydroxy, insbesondere Wasserstoff, bedeutet;

n eine ganze Zahl von 10 bis 40, insbesondere 12 bis 30, bedeutet;

m eine ganze Zahl von 1 bis 6, insbesondere 1, bedeutet;

U Hydroxy, Mercapto, $C_1$-$C_6$-Alkoxy, $C_1$-$C_6$-Alkyl, $NR^3R^4$ oder $NHR^3$, insbesondere Hydroxy oder $C_1$-$C_6$-Alkyl, bedeutet, worin

$R^3$ $C_1$-$C_8$-Alkyl, bevorzugt $C_1$-$C_4$-Alkyl, oder Methoxyethyl ist,

und B, W, V, Y, Y', X und Z die obengenannte Bedeutung haben.

Insbesondere bevorzugt sind Oligonucleotidanaloga der Formel I, worin V, Y'und Y die Bedeutung von Oxy haben.

Weiterhin besonders bevorzugt sind Oligonucleotidanaloga der Formel I, worin V, Y, Y' und W die Bedeutung von Oxy bzw. Oxo haben.

Ganz besonders bevorzugt sind Oligonucleotidanaloga der Formel I, worin V, Y, Y', W und U die Bedeutung von Oxy, Oxo bzw. Hydroxy haben.

Ferner sind Oligonucleotidanaloga der Formel I bevorzugt, worin $R^1$ für Wasserstoff steht.

Insbesondere bevorzugt sind Oligonucleotidanaloga der Formel I, worin U, V, W, X, Y' und Y die Bedeutung von Oxy, Oxo bzw. Hydroxy haben und $R^1$ gleich Wasserstoff ist.

Die wiederholt auftretenden Reste wie $R^2$, B, a, W, V, Y, U, $R^3$, $R^4$, p, q und Z können unabhängig voneinander gleiche oder verschiedene Bedeutungen haben, d.h. z.B. V bedeutet unabhängig voneinander Oxy, Sulfandiyl oder Imino.

Halogen steht vorzugsweise für Fluor, Chlor oder Brom.

Unter Heteroaryl ist der Rest eines monocyclischen oder bicyclischen ($C_3$-$C_9$)-Heteroaromaten zu verstehen, der im Ringsystem ein oder zwei N-Atome und/oder ein S- oder ein O-Atom enthält.

Als Beispiele für Gruppen $NR^3R^4$, in denen $R^3$ und $R^4$ zusammen mit dem sie tragenden Stickstoffatom einen 5- bis 6-gliedrigen heterocyclischen Ring bilden, der zusätzlich ein weiteres Heteroatom enthält, seien der Morpholinyl- und der Imidazolidinyl-Rest genannt.

Die Erfindung ist nicht auf α- und β-D- bzw. L-Ribofuranoside, α- und β-D- bzw. L-Desoxyribofuranoside und entsprechende carbocyclische Fünfringanaloga beschränkt, sondern gilt auch für Oligonucleotidanaloga, die aus anderen Zucker-Bausteinen aufgebaut sind, beispielsweise ringerweiterte und ringverengte Zucker, acyclische oder geeignete andersartige Zucker-Derivate. Die Erfindung ist ferner nicht auf die in Formel I beispielhaft aufgeführten Derivate des Phosphat-Restes beschränkt, sondern bezieht sich auch auf die bekannten Dephospho-Derivate.

Die Darstellung von Oligonucleotidanaloga der Formel I erfolgt ähnlich wie die Synthese biologischer Oligonucleotide in Lösung oder vorzugsweise an fester Phase, gegebenenfalls unter Zuhilfenahme eines automatischen Synthesegeräts.

Jedoch ist die Festphasen-Synthese von Oligonucleotiden mit einem Phosphatbzw. Phosphatester Rest am 3'-Ende nach der Standard-Phosphoramidit Chemie nach Caruthers (M. D. Matteucci and M. H. Caruthers, J. Am. Chem. Soc. 103, 3185 (1981)) nicht möglich, da der erste Nucleotid-Baustein über die 3'-Hydroxy-Gruppe an den festen Träger gebunden ist und daher aus diesen Synthesen stets Oligonucleotide mit einer 3'-Hydroxy-Gruppe resultieren. Es wurden verschiedene Verfahren nach der Festphasen-Methode beschrieben, die aber alle umständlich sind und womit sich oft keine Derivate wie Phosphatester oder Alkylphosphonate herstellen lassen (R. Eritja et al., Tetrahedron Lett. 32, 1511 (1991); P. Kumar et al., Tetrahedron Lett. 32, 967 (1991); W. T. Markiewcz und T.K. Wyrzykiewicz, Phoshorus, Sulfur and Silicon 51/52, 374 (1990); E. Felder et al., Tetrahedron Lett. 25, 3967 (1984); R. Lohrmann and J. Ruth, DNA 3, 122 (1984)).

Gegenstand der Erfindung ist daher ein Verfahren zur Herstellung der Oligonucleotidanaloga der Formel I, wobei

a) eine6 Nucleotideinheit mit 3'(2')-terminaler Phosphor(V)-Gruppierung und freier 5'-Hydroxy- oder Mercaptogruppe mit einer weiteren Nucleotideinheit mit 3'-ständiger Phosphor(III)- oder Phosphor(V)-Gruppierung oder deren aktivierten Derivaten umgesetzt wird, oder

b) das Oligonucleotidanalogon durch Fragmente in gleicher Weise aufgebaut wird,

in den nach (a) oder (b) erhaltenen Oligonucleotiden zum Schutz anderer Funktionen temporär eingeführte Schutzgruppen abgespalten werden und die so erhaltenen Oligonucleotidanaloga der Formel I gegebenenfalls in ihr physiologisch verträgliches Salz überführt werden.

Als Ausgangskomponente für die Festphasensynthese wird ein fester Träger der Formel IV eingesetzt

$$D\text{-}X'\text{-}CH_2CH_2\text{-}S(O)_x\text{-}CH_2CH_2\text{-}A\text{-}T \qquad (IV),$$

worin

A für einen Linker-Arm steht, der beispielsweise ein Rest einer Dicarbonsäure, eines Diols, eines Alkylamins, eines Dicarbonsäuremonoalkylamids, eines Säureamids oder eines Phosphats der Formel

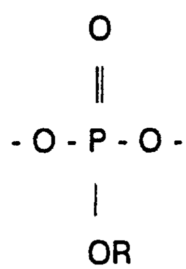

ist, worin R = Wasserstoff oder $C_1$-$C_6$-Alkyl, das gegebenenfalls durch -CN substituiert ist, vorzugsweise Methyl oder 2-Cyanoethyl, bedeutet, T ein fester Träger, beispielsweise aus Materialien wie CPG (Controlled Pore Glass), Kieselgel oder einem organischen Harz wie Polystyrol(PS) oder einem Pfropf-Copolymer aus PS und Polyethylenglykol(POE), ist, der durch funktionelle Gruppen wie Hydroxy, Amino, Halogen oder COOH in der Seitenkette modifiziert ist,
D für eine Schutzgruppe steht, die sich ohne Spaltung des Linkerarmes A und des X'-$CH_2CH_2$-$S(O)_x$-$CH_2CH_2$-Restes entfernen läßt (siehe Bioorg. Chem. 14 (1986) 274-325) wie 4-Methoxytetrahydropyranoyl und Dimethoxytrityl, vorzugsweise für Dimethoxytrityl, x eine ganze Zahl Null, 1 oder 2 ist und X' Oxy oder Sulfandiyl bedeutet.

Der Linkerarm A, der durch chemische Bindung (Amid, Ester u.a.) den festen Träger T mit dem schwefelhaltigen Rest verbindet (Damka et al., Nucleic Acids Res. 18, 3813 (1990)), ist vorzugsweise ein Bernsteinsäurerest (O-C(O)-$CH_2CH_2$-C(O)-), ein Oxalsäurerest, (O-C(O)-C(O)-), ein Alkylamin, vorzugsweise LCAA (Long Chain Alkyl Amin), oder Polyethylenglykol. Besonders bevorzugt ist ein Bernsteinsäurerest. In bestimmten Fällen, beispielsweise in Kombination mit Substituenten, die einer längeren Ammoniak-Behandlung nicht standhalten, sind labilere Linker wie der Oxalyl-Linker von Vorteil. Die Herstellung von festen Trägern der Formeln IV a-c ist in Beispiel 1 beschrieben.

| Träger | D | X' | x | A-T |
|---|---|---|---|---|
| IVa | DMTr | O | 2 | 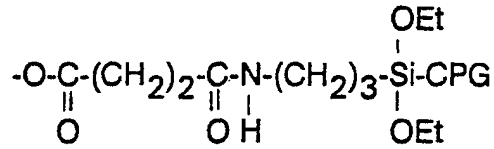 |
| IVb | DMTr | O | 2 | 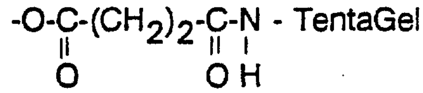 |

| Träger | D | X' | x | A-T |
|---|---|---|---|---|
| IVc | DMTr | O | 0 | 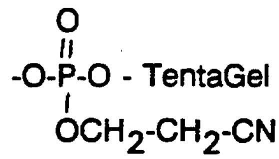 |

Die Festphasensynthese kann nach der Phosphattriester-Methode, der H-Phosphonat-Methode und der Phosphoramidit-Methode, vorzugsweise nach der Phosphoramidit-Methode (E. Sonveaux, Bioorg. Chem. 14, 274 (1986)), erfolgen. Zunächst wird stets die Schutzgruppe D , vorzugsweise durch eine Säure, beispielsweise Trichloressigsäure in Methylenchlorid, vom Träger der Formel IV abgespalten. Im Falle der Phosphoramidit-Methode wird der so erhaltene Träger der Formel IV'

$$HX'\text{-}CH_2\text{-}CH_2\text{-}S(O)_x\text{-}CH_2CH_2\text{-}A\text{-}T \qquad (IV'),$$

worin x, X', A und T die obengenannte Bedeutung haben, mit einem Nucleosid-Phosphoramidit der Formel V

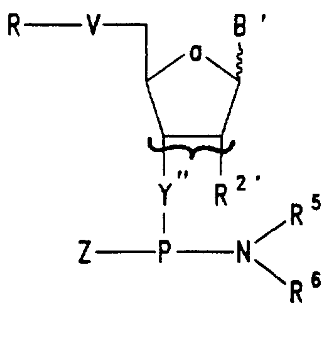

(V)

worin

| | |
|---|---|
| B' | B bedeutet, wobei gegebenenfalls in B vorhandene $NH_2$-Gruppen geschützt vorliegen können, |
| R | eine Schutzgruppe ist, die sich unter milden Bedingungen entfernen läßt wie 4-Methoxytetrahydropyranoyl oder Dimethoxytrityl, |
| $R^{2'}$ | Wasserstoff, $C_1$-$C_{18}$-Alkoxy, Halogen oder eine geschützte Hydroxy- oder Aminofunktion ist und |

| | |
|---|---|
| $R^5$ und $R^6$ | unabhängig voneinander $C_1$-$C_{12}$-Alkyl oder beide Reste zusammen einen 5 bis 6-gliedrigen Ring bilden, |
| Y" | Oxy, Sulfandiyl oder $(CH_2)_m$ ist und |
| a, m, V, und Z | die obengenannte Bedeutung haben, |

in Gegenwart einer schwachen Säure wie Tetrazol kondensiert. Anschließend oxidiert man den so erhaltenen Träger in an sich bekannter Weise mit Jodwasser (W=O) bzw. mit TETD (Tetraethylthiuramdisulfid) oder elementarem Schwefel, (W=S) bzw. mit Selen (W=Se) zum derivatisierten Träger der Formel VII

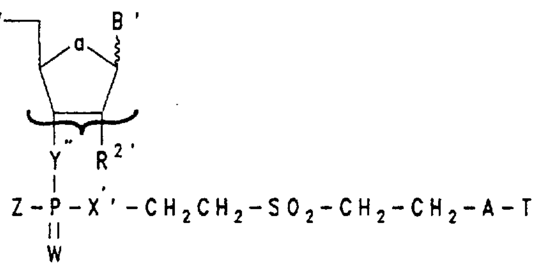

(VII)

worin

R, V, B', $R^{2'}$, Z, X', W, Y", A und T die obengenannte Bedeutung haben. Bevorzugt werden Träger der Formel VIIa

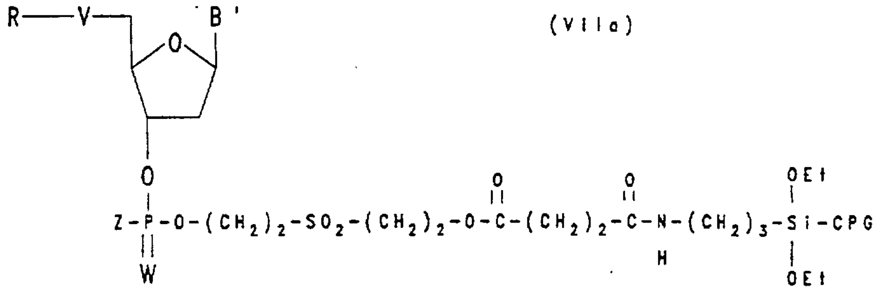

(VIIa)

hergestellt.

Das Phosphoramidit der Formel V kann beispielsweise aus dem Bis-Amidit der Formel VI

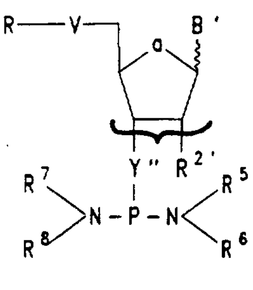

(VI)

worin

$R^7$ und $R^8$ gleich $R^5$ und $R^6$ sind und
a, R, V, B', $R^{2'}$, Y", $R^5$ und $R^6$ die obengenannte Bedeutung haben,

durch Umsetzung mit dem entsprechenden Alkohol oder Thioalkohol unter Tetrazol-Katalyse gewonnen werden (Beispiel 2, Methode A), wenn Z = Alkoxy oder Alkylmercapto ist (J. E. Marugg et al., Tedrahedron Lett. 27, 2271 (1986). Bevorzugte Bis-Amidite sind solche der Formel VIa

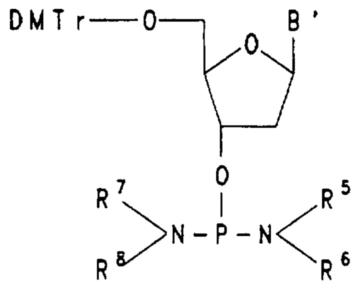

(VIa)

Auf diese Weise wurden beispielsweise die Amidite der Formeln VIII a-m hergestellt,

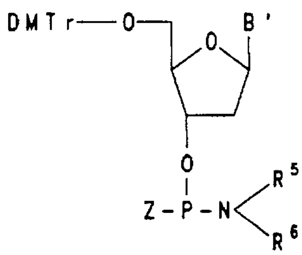

(VIII)

worin

$R^5$ und $R^6$ die obengenannte Bedeutung haben, Z die Bedeutung hat von

a) $O\text{-}CH_2CH_3$,
b) $O\text{-}i\text{-}C_3H_7$,
c) $O\text{-}n\text{-}C_6H_{13}$
d) $O\text{-}n\text{-}C_{18}H_{37}$,
e)

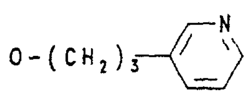

,

f)

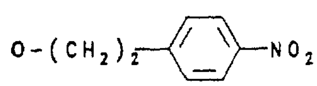

,

g-k) einem Rest der Formel III ($R^{11}$ = H), worin bei
g) p = 3 und q = 0,
h) p = 4 und q = 9,
i) p = 5 und q = 4 und bei
k) p = 8 und q = 13 ist,
p) $CH_3$
m)

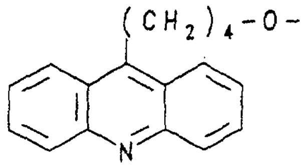

und B' bei

a), c) und d) $Cyt^{i\text{-}Bu}$, bei
b) und p) Thy und bei
e) - k) und m) $Cyt^{Bz}$ bedeutet.

Eine alternative Methode zur Beladung des Trägers ist die Umsetzung des Phosphitylierungsreagens der Formel IX

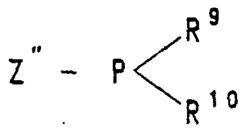

(IX),

worin

$R^9$ und $R^{10}$ unabhängig voneinander Cl,

oder Z" bedeuten, wobei Z" = Z ist, mit der Maßgabe, daß Hydroxy, Mercapto und SeH als geschützte Derivate vorliegen müssen, beispielsweise

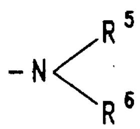

,

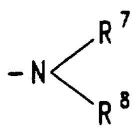

als $O\text{-}CH_2CH_2CN$, $O\text{-}CH_3$, $S\text{-}CH_2CH_2CN$,
$X'\text{-}CH_2CH_2\text{-}S(O)_{x'}\text{-}CH_2CH_2\text{-}X'\text{-}D$ oder

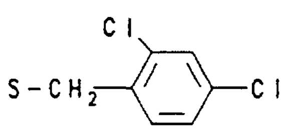

,vorzugsweise als $X'\text{-}CH_2CH_2\text{-}S(O)_{x'}\text{-}CH_2CH_2\text{-}X'\text{-}DMTr$, worin x' eine ganze Zahl Null oder 1 bedeutet, insbesondere als $O\text{-}CH_2CH_2\text{-}S\text{-}CH_2CH_2\text{-}O\text{-}DMTr$, und $R^5$, $R^6$, $R^7$, $R^8$, X', DMTr und D die obengenannte Bedeutung haben, mit einem Nucleosid mit freier 3'(2')-Funktion der Formel X

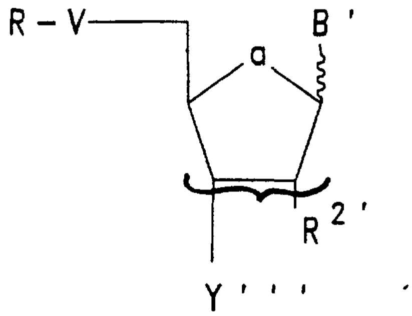

(X),

worin

Y''' = Oxy oder Sulfandiyl ist und V, B' und R die obengenannte Bedeutung haben, und anschließende Kondensation der so erhaltenen Verbindung an den Träger der Formel IV' in Gegenwart eines Kondensationsmittels, wie Tetrazol (für $R^9$, $R^{10}$ = $NR^5R^6$ bzw. $NR^7R^8$) oder Diisopropylamin (für $R^9$, $R^{10}$ = Cl), oft die schnellere Methode dar (Beispiel 3, Methode B). Anschließende Oxidation mit Jodwasser bzw. Schwefel oder Selen führt dann zur Verbindung der Formel VIIa. Nun kann die Schutzgruppe R entfernt werden und die Oligonucleotid-Synthese in bekannter Weise weitergeführt werden. Am Ende der Synthese werden von dem so erhaltenen trägergebundenen Oligonucleotidanalogon in bekannter Weise die Schutzgruppen entfernt und danach wird das erfindungsgemäße Oligonucleotidanalogon der Formel I vom Träger abgespalten.

Wurde die Synthese mit einem Baustein der Formel V im letzten Cyclus abgeschlossen, erhält man ein Oligonucleotidanalogon der Formel I ($R^1$=H) mit einer 5'-Hydroxy-Gruppe und einer phosphorhaltigen Konjugation am 3'-Ende. Setzt man dagegen im letzten Kondensationsschritt ein Phosphorylierungsreagenz, beispielsweise der Formel IX, worin $R^9$ = Z" ist, ein, dann resultiert aus der Synthese ein Oligonucleotidanalogon der Formel I mit $R^1$ = Formel II, das sowohl am 3'- als auch am 5'-Ende eine phosphathaltige Substitution aufweist.

Die Herstellung von Oligonucleotiden mit einer 3'-terminalen Phosphoramidat-Gruppe ist beispielsweise durch Umsetzung des Trägers der Formel IV' (x = 0) mit dem monomeren Methoxy-Phosphoramidit der Formel V ($Z=O-CH_3$) in Gegenwart von Tetrazol möglich, wenn man die Oxidation wie bei Jäger et al. (Biochemistry 27, 7237 (1988) beschrieben mit Jod/$H_2NR^3$ bzw. $HNR^3R^4$ ausführt, worin $R^3$ und $R^4$ die obengenannte Bedeutung haben.

In bestimmten Fällen ($Z=NHR^3$, $NR^3R^4$, O, S oder Se) kann die Einführung der Gruppe Z auch nach der H-Phosphonat-Methode erfolgen, indem man zunächst ein Nucleosid-H-Phosphonat der Formel XI

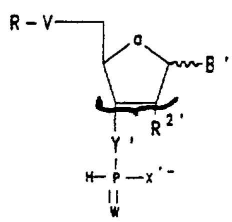

(IX)

worin R, V, a, B', Y', X' und W die obengenannte Bedeutung haben, in Gegenwart eines Kondensationsmittels wie Pivaloyl- oder Adamantoylchlorid und einer Base wie Pyridin, mit einem Träger der Formel IV' umsetzt. Der gebildete H-Phosphonat-Diester der Formel VII'

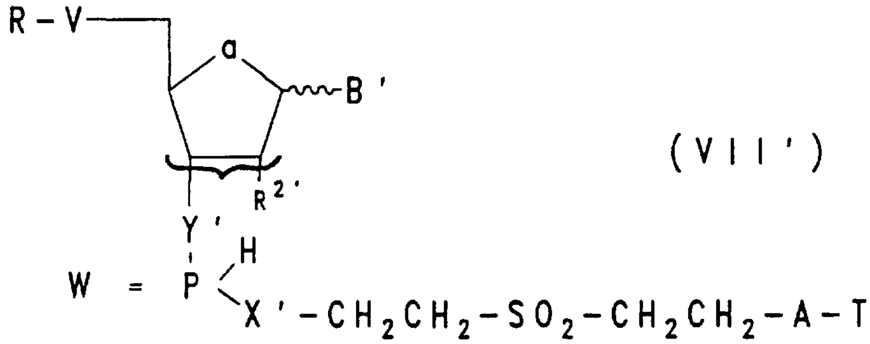

(VII')

wird dann einer oxidativen Phosphoramidierung (B. Froehler, Tetrahedron Lett. 27, 5575 (1986) bzw. einer Jodwasser-, Schwefel- oder Selenoxidation unterworfen. Auf diese Weise läßt sich zum Beispiel ausgehend von VII' (x = 0) mit Cholesteryl-oxycarbonyl-aminoalkylamin in Gegenwart von Tetrachlorkohlenstoff ein Oligonucleotid mit einer 3'-terminalen Cholesteryl-Gruppe herstellen. Durch oxidative Amidierung mit 2-Methoxyethylamin erhält man beispielsweise Oligonucleotide mit einem 3'-O-(2'-Methoxyethyl)-phosphoramidat-Rest. Der weitere Kettenaufbau erfolgt dabei in bekannter Weise nach der Phosphoramidit-, H-Phosphonat- oder Triester-Methode.

Die Herstellung von Oligonucleotidanaloga der Formel I ist ferner nach der Triester-Methode möglich, indem man die Hydroxygruppe des Trägers der Formel IV' mit einem geschützten Phosphatdiester der Formel XII

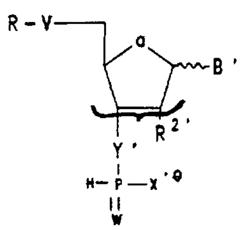

(XII)

worin R, V, a, B', $R^2$, Y', Z, W und X' die obengenannte Bedeutung haben, in Gegenwart eines Kondensationsmittels umsetzt. Bevorzugte Kondensationsreagenzien sind Arylsulfonsäurechloride wie Mesitylen-, 2.4.6-Triisopropylbenzol- oder 8-Chinolinsulfonsäurechlorid in Gegenwart nucleophiler Katalysatoren wie Imidazol, Triazol, Tetrazol oder deren substituierte Derivate wie N-Methylimidazol, 3-Nitrotriazol oder 5-(p-Nitrophenyl)-tetrazol. Besonders bevorzugte Kondensationsmittel sind 4-substituierte Derivate von Pyridin-N-oxid oder Chinolin-N-oxid (Efimov et al., Nucleic Acids Research 13 (1985) 3651). Das Triester-Verfahren hat gegenüber dem H- Phosphonat- und Phosphoramidit-Verfahren den Vorteil, daß kein zusätzlicher Oxidationsschritt erforderlich ist.

Wird die Oligonucleotid-Synthese mit einem Sulfandiyl- (x=0) oder Sulfinyl- (x=1) Träger der Formel IV' durchgeführt, so werden diese Gruppen am Ende in an sich bekannter Weise [Funakoshi et al. Proc. Natl. Acad. Sci. 88 (1991), 6982] zum Sulfonyl-Rest oxidiert, um eine leichte Spaltung mit Basen, vorzugsweise Ammoniak, zu gewähren.

Die Art der Amino-Schutzgruppen der Basen B' und die Beschaffenheit des Linkerarmes A richten sich im Einzelfall nach der Art des Substituenten Z, da diese nach vollständiger Synthese problemlos spaltbar sein müssen. Zum Beispiel kann man bei der Herstellung eines Oligonucleotid-3'-Phosphatisopropylesters (Z=O-i-$C_3H_7$), für B= Ade und Cyt mit den Benzoyl- (Bz) bzw. für B= Gua mit den Isobutyryl- (i-Bu) Schutzgruppen arbeiten. Dagegen verwendet man zur Synthese eines Oligonucleotid-3'-methylphosphonatesters (Z=$CH_3$) oder Ethylesters (Z=O-$C_2H_5$) für B= Ade und Gua bevorzugt die labileren Phenoxyacetyl (PAC) bzw. für B= Cyt die iso-Butyryl-Schutzgruppen.

Manche Konjugate besitzen zusätzliche funktionelle Gruppen, die vor dem Einbau in die monomeren Bausteine der Formel V in geeigneter Weise geschützt werden müssen. Beispielsweise ist die Carboxyl-Gruppe des Fluoresceins als Alkylester zu schützen. Im Psoralen kann die Amid-Gruppe als N-Fmoc (Fluorenylmethoxycarbonyl) geschützte Verbindung vorliegen. Hydroxygruppen können durch Acylierung oder Silylierung (t-Butyldimethylsilyl) vor Nebenreaktionen geschützt werden. Aminogruppen können auch Trifluoracetyl-geschützt vorliegen. In Ausnahmefällen können die Konjugate so wenig stabil sein, daß sie unter den Bedingungen der Schutzgruppen-Abspaltung der Oligonucleotid-Synthese bereits zerstört würden. In solchen Fällen ist es günstig, im Monomer der Formel V lediglich einen Linker-Arm mit einer funktionellen Gruppe, beispielsweise Z = HN-$(CH_2)_x$-NH-Fmoc, worin x eine ganze Zahl von 2-12, bevorzugt 4-6, bedeutet, einzubauen. Nach Einbau in das Oligonucleotid und Entfernung der Schutzgruppen,

vorzugsweise mit Ammoniak, kann die freie Aminogruppe mit Aktivestern gekoppelt werden. So wurde beispielsweise der basenlabile Acridinium-Ester hergestellt.

Die Charakterisierung der synthetisierten Oligonucleotid-Derivate erfolgt durch Elektro-Spray-Ionisations Massenspektrometrie (Stults u. Masters, Rapid Commun. Mass. Spectr. 5 (1991) 350).

Die erfindungsgemäßen Oligonucleotidanaloga der Formel I wurden auf ihre Stabilität im Serum und gegenüber bekannten Exonucleasen getestet.

Überraschenderweise wurde gefunden, daß alle Oligonucleotidanaloga der Formel I im Vergleich zu den nicht modifizierten Oligonucleotiden eine stark erhöhte Stabilität gegenüber den Nucleasen des Serums aufweisen, während ihr Hybridisationsverhalten nur wenig beeinflußt wird.

Während nicht modifizierte Oligonucleotide in fötalem Kälberserum eine Halbwertszeit von etwa zwei Stunden aufweisen, sind alle Oligonucleotidanaloga der Formel I etwa 16 Stunden ausreichend stabil. Die Oligonucleotidanaloga der Formel I sind darüberhinaus stabil gegenüber Schlangengift-Phosphodiesterase, während gegen Milz-Phosphodiesterase nur solche mit $R^1$ ungleich Wasserstoff resistent sind. Nicht modifizierte Oligonucleotide werden von Schlangengift-Phosphodiesterase vom 3'-Ende und von Milz-Phosphodiesterase vom 5'-Ende exonucleolytisch abgebaut.

Die Oligonucleotidanaloga der Formel I bilden aufgrund der Watson-Crick-Basenpaarung mit komplementären einzelsträngigen Nucleotid-Sequenzen stabile doppelsträngige Hybride, während sie mit doppelsträngigen Nucleinsäuren aufgrund der Hoogsteen-Basenpaarung tripelhelicale Strukturen ausbilden. Dadurch ist die Regulation oder Unterdrückung biologischer Funktionen von Nucleinsäuren mit den erfindungsgemäßen Oligonucleotidanaloga möglich, beispielsweise die Unterdrückung der Expression zellulärer Gene wie auch der von Onkogenen oder von viralen Genom-Funktionen. Oligonucleotidanaloga der Formel I sind daher verwendbar als Heilmittel zur Therapie oder Prophylaxe viraler Infektionen oder Krebskrankheiten.

Die Wirksamkeit der erfindungsgemäßen Oligonucleotide wurde anhand der Inhibition der Virus-Vermehrung von HSV-1 nachgewiesen. Als gegen HSV-1 wirksame Oligonucleotide der Formel 1 wurden beispielsweise gefunden:

| Sequenz | Angriffspunkte bei HSV-1 |
|---|---|
| 5' GGG GCG GGG CTC CAT GGG GG | IE 110 (Start) |
| 5' CCG GAA AAC ATC GCG GTT GT | UL 30 (Mitte) |
| 5' GGT GCT GGT GCT GGA CGA CA | UL 48 (Mitte) |
| 5' GGC CCT GCT GTT CCG TGG CG | UL 52 (Mitte) |
| 5' CGT CCA TGT CGG CAA ACA GCT | UL 48 (Start) |
| 5' GAC GTT CCT CCT GCG GGA AG | IE4/5 (splice site) |

Die ausgewählten Sequenzen sind in ihrer natürlichen Form, also ohne 3'-Derivatisierung, gegenüber HSV-1 in Zellkultur unwirksam, wahrscheinlich weil sie einem raschen Abbau im Serum unterliegen oder weil ihre Zellgängigkeit zu gering ist. Dagegen hemmen die 3'-derivatisierten Oligonucleotide der Formel I die HSV-1 Vermehrung in unterschiedlichem Maße. Als antiviral unwirksame Kontroll-Sequenzen mit der entsprechenden chemischen Derivatisierung dienten:

| | |
|---|---|
| 5' CCA GGG TAC AGG TGG CCG GC | Kontrolle |
| 5' GAC TAA TCG GGA ATG TTA AG | Kontrolle |

Ein mit Psoralen am 3'-Ende modifiziertes Oligonucleotid der Formel I (Beispiel 4s) erkennt die IE4/5 Region von HSV-2 und hemmt die Replikation von HSV-2. Die antivirale Wirksamkeit der Psoralen-Konjugate kann durch Bestrahlung mit UV-Licht deutlich gesteigert werden. Das HSV-1/2 Genom mit seinen 160 000 Basen bietet natürlich unzählig viele andere Zielsequenzen unterschiedlicher Effizienz zur Inhibition der Virus-Vermehrung an. Durch Variation der Nucleotid-Sequenzen läßt sich das therapeutische Prinzip auch auf beliebige andere Viren, Bakterien oder sonstige Pathogene anwenden. Voraussetzung für eine Übertragung auf andere Krankheitsauslöser ist lediglich, daß

man die für den Lebenscyclus essentiellen Gene dieser Krankheitserreger kennt. Diese sind in ihrer Sequenz in großer Vielfalt in den sogenannten Gen-Datenbanken niedergelegt. Ähnliches gilt für Onkogene oder andere zelluläre Gene, die in ihrer Funktion unterdrückt werden sollen. Beispiele für andere zelluläre Gene sind solche, die für Enzyme, Rezeptoren, Ionenkanäle, Immunmodulatoren, Wachstumsfaktoren oder andere regulatorischen Proteine codieren. Beispiele für Onkogene sind abl, neu, myc, myb, ras, fos, mos, erbB, ets, jun, p53, src und rel.

Antisense bzw. Triplex Forming Oligonucleotidsequenzen sind beispielsweise als Inhibitoren der cyclo-AMP-abhängigen Protein-Kinase (L. Sheffield, Exp. Cell Res. 192 (1991), 307), des Strychnin-sensitiven Glycin Rezeptors (Akagi et al., Proc. Natl. Acad. Sci. USA 86 (1989) 86, 8103), des Chlorid-Kanals (Sorscher et al., Proc. Natl. Acad. Sci. USA 88 (1991), 7759), des Interleukins-6 (Levy et al., J. Clin. Invest. 88 (1991), 696), des basischen Fibroblasten-Wachstumsfaktors (Becker et al., EMBO J. 8 (1989), 3685) und des c-myc Onkogens (Postel et al., Proc. Natl. Acad. Sci. USA 88 (1991), 8227) bekannt. Weitere Sequenz-Beispiele für andere Ziel-Moleküle sollen die breite Anwendbarkeit der erfindungsgemäßen Oligonucleotide verdeutlichen.

a) Antisense Oligonucleotide gegen HIV-1:

5' ACA CCC AAT TCT GAA AAT GG 3' (splice site)
5' AGG TCC CTG TTC GGG CGC CA 3' (primer binding site)

b) EGF Receptor (Epidermal Growth Factor Receptor)

5' GGG ACT CCG GCG CAG CGC 3' (5'-untranslated)

5' GGC AAA CTT TCT TTT CCT CC 3' (aminoterminal)

c) p53 Tumorsuppressor

5' GGG AAG GAG GAG GAT GAG G 3' (5'-noncoding)
5' GGC AGT CAT CCA GCT TCG GAG 3' (start of translation)

d) c-fos oncogen

5' CCC GAG AAC ATC ATG GTC GAA G 3' (start of translation)
5' GGG GAA AGC CCG GCA AGG GG 3' (5'-noncoding)

e) ELAM-1 (Endothelial Leucocyte Adhesion Molecule)

5' ACT GCT GCC TCT TGT CTC AGG 3' (5'-noncoding)
5' CAA TCA ATG ACT TCA AGA GTT C 3' (start of translation)

f) ICAM-1 (Intracellular Adhesion Molecule)

5' CTC CCC CAC CAC TTC CCC TC 3' (3'-untranslated)
5' GCT GGG AGC CAT AGC GAG G 3' (start of translation)

g) BCR-ABL (Philadelphia chromosome translocation)

5' GCT GAA GGG CTT CTT CCT TAT TG 3' (BCR-ABL breakpoint)

DNA-Sonden aus Oligonucleotidanaloga der Formel I bieten gegenüber den literaturbekannten Oligonucleotid-Derivaten mit einer 3'-Hydroxygruppe einerseits den Vorteil erhöhter Nucleasestabilität, andererseits gestatten sie aber auch die Aufnahme gleicher oder unterschiedlicher Marker-Moleküle an beiden Enden des Oligonucleotids. Von Vorteil ist, daß verschiedenene Marker-Gruppierungen innerhalb eines Oligonucleotids selektiv anregen lassen (Doppelmarkierung). Die bifunktionelle Derivatisierung kann auch dazu verwendet werden, um am einen Ende einen Marker und am anderen Ende eine zusätzliche Funktion (beispielsweise Affinitäts-Label) einzuführen. Zu diesem Zweck kann beispielsweise am 3'-Ende des Oligonucleotids Biotin eingebaut werden, das Avidin oder Streptavidin erkennt, sowie am 5'-Ende über einen Alkylamino-Linker ein Acridiniumester Chemilumineszenz-Marker angebracht werden.

In vielen Fällen ist zudem das Penetrationsverhalten der erfindungsgemäßen Oligonucleotidanaloga günstiger als bei den nichtmodifizierten Oligonucleotiden, insbesondere wenn lipophile Reste eingeführt wurden. Die erhöhte Stabilität der Oligonucleotide sowie ihre verbesserte Zellgängigkeit kommt in einer im Vergleich zu den nichtmodifizierten Oligonucleotiden höheren biolgischen Aktivität zum Ausdruck.

Die zuvor genannten diagnostischen, prophylaktischen und therapeutischen Anwendungen der erfindungsgemäßen Oligonucleotidanaloga stellen nur eine Auswahl an repräsentativen Beispielen dar und ihre Verwendung ist daher nicht darauf beschränkt. Darüber hinaus können die erfindungsgemäßen Oligonucleotidanaloga beispielsweise auch als Hilfsmittel in der Biotechnologie und Molekularbiologie eingesetzt werden.

Die Erfindung betrifft weiterhin pharmazeutische Zubereitungen, die eine wirksame Menge einer oder mehrerer Verbindungen der Formel I oder deren physiologisch verträglichen Salze, gegebenenfalls zusammen mit physiologisch verträglichen Hilfsund/oder Trägerstoffen und/oder zusammen mit anderen bekannten Wirkstoffen, enthalten sowie ein Verfahren zur Herstellung dieser Zubereitungen, dadurch gekennzeichnet, daß man den Wirkstoff, zusammen mit dem Träger und eventuell weiteren Hilfs-, Zusatz- oder Wirkstoffen in eine geeignete Darreichungsform bringt. Die Applikation erfolgt vorzugsweise intravenös, topisch oder intranasal.

Beispiel 1: Herstellung eines Trägers der Formel IV

a) Herstellung des Trägers der Formel IVa durch Umsetzung von Aminopropyl-CPG mit dem Succinat des Bishydroxyethylsulfon-dimethoxytritylethers
4.56 g des einfachen Dimethoxytrityl(DMTr)-ethers von Bis-(2-Hydroxyethyl)-sulfon (10 mmol) werden durch zweimaliges Aufnehmen und Einengen in abs. Pyridin getrocknet, in 25 ml abs. Pyridin gelöst, mit 1.78 g (14 mmol) DMAP (Dimethylaminopyridin) und 1.4 g Bernsteinsäureanhydrid (14 mmol) versetzt und diese Mischung 3 Stunden bei Raumtemperatur gerührt. Nach vollständiger Reaktion wird eingeengt, der Rückstand zur Entfernung des Pyridins dreimal in Toluol aufgenommen und eingeengt und danach in 220 ml Methylenchlorid aufgenommen. Die organische Phase wird mit 10%-iger Zitronensäure (110 ml) und 3-mal mit 110 ml Wasser gewaschen, über Natriumsulfat getrocknet und eingeengt. Der resultierende feste Rückstand wird im Vakuum getrocknet (5.64 g). Von diesem Succinat werden 1.67 g (3 mmol) zweimal in Pyridin abs. aufgenommen und eingeengt und in einer Mischung aus 0.65 ml Pyridin abs. und 6 ml Tetrahydrofuran (THF) abs. gelöst. Dann gibt man eine Lösung aus 420 mg (3 mmol) p-Nitrophenol und 687 mg DCC (Dicyclohexylcarbodiimid, 3.3 mmol) in 2.1 ml THF abs. zu und rührt zwei Stunden bei Raumtemperatur. Nach vollständiger Umsetzung wird vom ausgefallenen Dicyclohexylharnstoff abzentrifugiert. Das Sediment wird in 1 ml abs. Ether aufgeschlämmt und nochmals zentrifugiert. 1.5 g des Aminopropyl-CPG Trägers der Firma Fluka (500 Å, 100 µmol/g Aminofunktion) werden in einer Mischung aus 1.8 ml DMF abs. und 350 µl Triethylamin aufgeschlämmt, mit den vereinigten vom Sediment abdekantierten Lösungen des Succinat-nitrophenylesters versetzt und 16 Stunden bei Raumtemperatur geschüttelt. Der feste Träger wird abgetrennt und zur Blockierung reaktiver Gruppen mit 3 ml Verkappungsreagenz (Acetanhydrid / 2.6-Lutidin / DMAP; je 0.25 M in THF) eine Stunde bei Raumtemperatur geschüttelt. Dann wird der derivatisierte CPG-Träger abgesaugt, mit Methanol, THF, Methylenchlorid und Ether gewaschen und anschließend im Vakuum bei 40°C getrocknet. Die Beladung des Trägers der Formel IVa mit Dimethoxytrityl-haltiger Komponente beträgt 38 µmol/g.

b) Herstellung des Trägers der Formel IVb durch Umsetzung von TentaGel®
(®= eingetragenes Warenzeichen der Fa. Rapp, Tübingen) mit dem Succinat des Bishydroxyethylsulfon-dimethoxytritylethers

Die Amino-Form des TentaGel-Harzes, einem PS/POE-Copolymer mit 250 µmol/g Aminofunktion (100 mg), werden in einer Mischung aus 360 µl DMF und 70 µl Triethylamin aufgeschlämmt, mit 400 µmol des Succinat-p-nitrophenylesters (Herstellung s. Bsp. 1a) versetzt und 16 Stunden bei Raumtemperatur geschüttelt. Anschließend wird wie in Bsp. 1a) beschrieben aufgearbeitet. Die Beladung des TentaGel-Harzes der Formel IVb mit Dimethoxytrityl-haltiger Komponente beträgt 98 µmol/gr.

c) Herstellung des Trägers IVc durch Umsetzung von TentaGel (Hydroxyform) mit dem Phosphitylierungsreagenz der Formel IX (Z" = DMTr-O-$CH_2CH_2$-S-$CH_2CH_2$-O-;
$R^9$ = N(i-$C_3H_7$)$_2$; $R^{10}$ = O-$CH_2CH_2$CN).
Die Hydroxy-Form des TentaGel-Harzes mit 500 µmol/gr Hydroxyfunktion (50 mg) werden mit 10 Äquivalenten des Phosphitylierungsreagenz der Formel IX (Z" = DMTr-O-$CH_2CH_2$-S-$CH_2CH_2$-O-, $R^9$ = N(i-$C_3H_7$)$_2$; $R^{10}$ = O-$CH_2CH_2$CN) in Gegenwart von 25 Äquivalenten Tetrazol in Acetonitril bei 22°C umgesetzt. Nach der Oxidation mit Jodwasser (1,3 g Jod in THF/Wasser/Pyridin; 70:20:5 =v:v:v) wird wie in Beispiel 1a aufgearbeitet. Die Beladung des Trägers der Formel IVc mit Dimethoxytrityl-haltiger Komponente beträgt 247 µmol/gr.

Beispiel 2: Herstellung geschützter Nucleosid-3'-Phosphoramidite der Formel VIII

a) Herstellung von VIII a (B'= $Cyt^{iBu}$, Z=O-$CH_2CH_3$, $R^5$=$R^6$=i-$C_3H_7$)
2 mmol des Nucleosid-3'-phosphorbisamidits der Formel VI (B' = $Cyt^{iBu}$, $R^5$=$R^6$=$R^7$=$R^8$=i-$C_3H_7$) werden zweimal in 20 ml Acetonitril abs. aufgenommen und eingesetzt und in 20 ml Acetonitril abs. gelöst. Dann tropft man eine Lösung aus 2.4 mmol Ethanol und 1.2 mmol sublimiertem Tetrazol in 5 ml abs. Acetonitril während 15 Minuten zu. Nachdem noch 2.5 Stunden weitergerührt wurden, verdünnt man mit 75 ml Methylenchlorid und extrahiert die organische Phase mit 50 ml 5%-iger Natriumhydrogencarbonat-Lösung. Die wässrige Lösung wird 2-mal mit 50 ml Methylenchlorid gewaschen, die vereinigten organischen Phasen über Natriumsulfat getrocknet und im Vakuum konzentriert. Zur Reinigung wird der Rückstand über eine Kieselgelsäule mit Methylenchlorid/n-Heptan/Triethylamin (45:45:10; v:v:v) chromatographiert. Man erhält 0.7 g der gewünschten diastereomeren Substanz als dünnschichtchromatographisch einheitliche Verbindung. ($^{31}$P-NMR σ=146.7, 147.5 ppm). Als Nebenprodukt lassen sich Spuren des entsprechenden Bis-ethyl-phosphits isolieren ($^{31}$P-NMR σ=139.3 ppm).

b) Herstellung von VIII b (B'= Thy, Z=O-i-$C_3H_7$, $R^5$=$R^6$=i-$C_3H_7$)
Die Herstellung erfolgt durch Phosphitylierung des 5'-O-Dimethoxytrityl-thymidins der Formel X (B'= Thy (ß-Stellung); R= DMTr, V = 0, a=0, Y"=0, 2mmol) mit dem Bisamidit der Formel IX (Z"=O-i-$C_3H_7$, $R^9$=$R^{10}$=N(i-$C_3H_7$)$_2$; 4 mmol) in Gegenwart von Tetrazol (0.5 mmol) in 10 ml abs. Methylenchlorid. Der Ansatz wird wie in Beispiel 2a aufgearbeitet. ($^{31}$P-NMR σ=145.04 ppm, 145.66 ppm)

c) Herstellung von VIII c (B'= $Cyt^{iBu}$, Z=-O-n-$C_6H_{13}$, $R^5$=$R^6$=i-$C_3H_7$)
Analog Beispiel 2a aus dem Bisamidit der Formel VIa (B'= $Cyt^{iBu}$,
$R^5$=$R^6$=$R^7$=$R^8$=i-$C_3H_7$) durch Umsetzung mit einem Äquivalent n-Hexanol unter Tetrazol-Katalyse. ($^{31}$P-NMR 148.1 ppm, 148.5 ppm).

d) Herstellung von VIII d (B'= $Cyt^{i\text{-}Bu}$, Z=-O-n-$C_{18}H_{37}$, $R^5$=$R^6$=i-$C_3H_7$)
Analog Beispiel 2a aus dem Bisamidit der Formel VIa (B'= $Cyt^{i\text{-}Bu}$, $R^5$=$R^6$=$R^7$=$R^8$=i-$C_3H_7$) durch Umsetzung mit einem Äquivalent n-Octadecanol unter Tetrazol-Katalyse. ($^{31}$P-NMR 147.2 ppm, 147.9 ppm).

e) Herstellung von VIII e (B'= $Cyt^{Bz}$, Z = 3-Pyridylpropan-3-oxy,
$R^5$=$R^6$=$R^7$=$R^8$=i-$C_3H_7$).
Analog Beispiel 2a aus dem Bisamidit der Formel VIa (B'= $Cyt^{Bz}$,
$R^5$=$R^6$=$R^7$=$R^8$=i-$C_3H_7$, $R^{2'}$= H) durch Umsetzung mit einem Äquivalent 3-Pyridin-(propan-3-ol) unter Tetrazol-Katalyse. In diesem Fall konnten die beiden Diastereomeren säulenchromatographisch getrennt werden. ($^{31}$P-NMR Diastereomer 1: 147.7 ppm, Diastereomer 2: 148.2 ppm)

f) Herstellung von VIII f (B'= $Cyt^{Bz}$, Z= p-Nitrophenylethyl-2-oxy, $R^5$=$R^6$=i-$C_3H_7$).
Analog Beispiel 2a aus dem Bisamidit der Formel VIa (B'= $Cyt^{Bz}$,
$R^5$=$R^6$=$R^7$=$R^8$=i-$C_3H_7$) durch Umsetzung mit einem Äquivalent p-Nitrophenyl-ethan-2-ol unter Tetrazol-Katalyse. ($^{31}$P-NMR 148.1 ppm, 148.6 ppm).

g) Herstellung von VIII g (B'= $Cyt^{Bz}$, Z=-$(OCH_2CH_2)_3OCH_3$, $R^5$=$R^6$=i-$C_3H_7$) Analog Beispiel 2a aus dem Bisamidit der Formel VIa (B'= $Cyt^{Bz}$, $R^5$ =$R^6$ =$R^7$ =$R^8$ =i-$C_3H_7$) durch Umsetzung mit einem Äquivalent Triethylenglycol-monomethylether unter Tetrazol-Katalyse. ($^{31}$P-NMR 148.5 ppm, 148.9 ppm).

h) Herstellung von VIII h (B'= $Cyt^{Bz}$, Z=-$(OCH_2CH_2)_4O(CH_2)_9CH_3$, $R^5$ =$R^6$ =i-$C_3H_7$)
Analog Beispiel 2a aus dem Bisamidit der Formel VIa (B'= $Cyt^{Bz}$, $R^5$=$R^6$=$R^7$=$R^8$=i-$C_3H_7$) durch Umsetzung mit einem Äquivalent Tetraethylenglycol-monodecylether unter Tetrazol-Katalyse. ($^{31}$P-NMR 148.4 ppm, 148.8 ppm).

i) Herstellung von VIII i (B'= $Cyt^{Bz}$, Z=-$(OCH_2CH_2)_5O(CH_2)_4CH_3$, $R^5$=$R^6$=i-$C_3H_7$)
Analog Beispiel 2a aus dem Bisamidit der Formel VIa (B'= $Cyt^{Bz}$, $R^5$=$R^6$=$R^7$=$R^8$=i-$C_3H_7$) durch Umsetzung mit einem Äquivalent Pentaethylenglycol-monopentylether unter Tetrazol-Katalyse. ($^{31}$P-NMR 148.4 ppm, 148.9 ppm).

k) Herstellung von VIII k (B'= $Cyt^{Bz}$, Z=-$(OCH_2CH_2)_8O(CH_2)_{13}CH_3$, $R^5$=$R^6$=i-$C_3H_7$)
Analog Beispiel 2a aus dem Bisamidit der Formel VIa (B'= $Cyt^{Bz}$, $R^5$ =$R^6$ =$R^7$ =$R^8$ =i-$C_3H_7$) durch Umsetzung mit einem Äquivalent Octaethylenglycol-monotetrdecylether unter Tetrazol-Katalyse. ($^{31}$P-NMR 148.4 ppm, 148.8 ppm).

l) Herstellung von VIII p (B'= Thy, Z=$CH_3$, $R^5$=$R^6$=i-$C_3H_7$)
Analog Beispiel 2b aus 5'-O-Dimethoxytritylthymidin durch Phosphitylierung mit dem Reagenz der Formel IX (Z"=$CH_3$, $R^9$=Cl, $R^{10}$=N(i-$C_3H_7)_2$, wobei anstelle von Tetrazol mit zwei Äquivalenten Diisopropylethylamin katalysiert wird. ($^{31}$P-NMR 120.6 ppm, 121.0 ppm).

m) Herstellung von VIII m (B'= $Cyt^{Bz}$, Z= Acridin-9-(butyl-4-oxy)-, $R^5$=$R^6$=i-$C_3H_7$)
Analog Beispiel 2a aus dem Bisamidit der Formel VIa (B'= $Cyt^{Bz}$, $R^5$=$R^6$=$R^7$=$R^8$=i-$C_3H_7$) durch Umsetzung mit einem Äquivalent 9-(4-Hydroxybutyl)acridin unter Tetrazol-Katalyse. ($^{31}$P-NMR 146.7 ppm, 147.4 ppm).

Beispiel 3: Herstellung des trägergebundenen Nucleotids der Formel VII

a) Methode A: Herstellung eines Trägers der Formel VIIa-1 durch Kopplung des Nucleosid-3'-phosphoramidits der Formel VIIIb
7.5 mg des Trägers aus Beispiel 1a, der 0.2 µmol des Bishydroxyethylsulfon-dimethoxytritylethers gebunden hält, werden mit 3 % Trichloressigsäure behandelt, wobei die DMTr-Schutzgruppe abgespalten wird, mit Acetonitril gewaschen, und anschließend mit 2 µmol des Nucleosid-3'-phosphoramidits der Formel VIIIb (B'= Thy, Z=O-i-$C_3H_7$, $R^5$=$R^6$=i-$C_3H_7$) in Gegenwart von Tetrazol (10 µmol) in Acetonitril zur Reaktion gebracht. Die Reaktionszeit beträgt 2.5 Minuten. Danach wird mit Jod (für W=O; 1.3 g Jod in THF/Wasser/Pyridin; 70:20:5=v:v:v, 5 Minuten) oxidiert.

b) Methode B: Herstellung eines Trägers der Formel VIIa-2 durch Reaktion über das Phosphitylierungsreagenz der Formel IX
Das Phosphitylierungsreagenz der Formel IX (Z"=n-Octyl, $R^9$=$R^{10}$=Cl; 1 Äquivalent) wird in Gegenwart von 1.2 Äquivalenten Diisopropylethylamin (DIPEA) in abs. Acetonitril oder Methylenclorid mit einem Nucleosid der Formel X (1 Äquivalent 5'-O-Dimethoxytritylthymidin, B' = β-Stellung, Y''' = 0,) bei -78°C zum entsprechenden Nucleosid-3'-O-n-octylphoshonmonochlorid umgesetzt. Der Träger der Formel IVa wird zur Abspaltung der Schutzgruppe D=DMTr wie in Methode A beschrieben behandelt, mit Acetonitril gewaschen und in Gegenwart von DIPEA mit einem Überschuß des in situ zubereiteten Nucleosid-3'-O-n-octylphoshonmonochlorids umgesetzt. Nach Oxidation mit Jodwasser erhält man ein trägergebundenes Nucleotid der Formel VIIa-2, das für die weitere Oligonucleotid-Synthese zur Verfügung steht.

Beispiel 4: Herstellung von Oligonucleotiden der Formel I

Das Monomer ist jeweils ein ß-D-Desoxyribonucleosid)

a) Herstellung eines Oligonucleotids der Formel I a ($R^1$=$R^2$=H, Z=O-i-$C_3H_7$, a=U=V=W=X=Y=Y'=O, B=Thy, n=9)

TpTpTpTpTpTpTpTpTpTp-(O-i-$C_3H_7$)

0.2 μmol des Trägers VIIa-1 (B'=Thy, W=O, Z=O-i-$C_3H_7$) aus Beispiel 3a werden nacheinander mit folgenden Reagenzien behandelt:

1. Acetonitril abs.
2. 3 % Trichloressigsäure in Dichlormethan
3. Acetonitril abs.
4. 4 μmol 5'-O-Dimethoxytritylthymidin-3'-phosphorigsäure-ß-cyanoethylesterdiisopropylamidit und 25 μmol Tetrazol in 0.15 ml Acetonitril abs.
5. Acetonitril
6. 20 % Acetanhydrid in THF mit 40% Lutidin und 10 % Dimethylaminopyridin
7. Acetonitril
8. Jod (1.3 gr in THF/Wasser/Pyridin; 70:20:5=v:v:v)

Die Schritte 1 bis 8, nachfolgend ein Reaktionszyklus genannt, werden zum Aufbau des Decathymidylat-Derivats 8-mal wiederholt. Nach abgeschlossener Synthese erfolgt die Abspaltung der Dimethoxytritylgruppe wie in den Schritten 1 bis 3 beschrieben. Durch 1.5-stündige Behandlung mit Ammoniak wird das Oligonucleotid vom Träger gespalten und zugleich werden die ß-Cyanoethyl-Gruppen eliminiert. Da das Oligonucleotid keine Amino-Schutzgruppen enthält, ist keine weitere Ammoniak-Behandlung notwendig. Das erhaltene Rohprodukt an Decathymidylat-3'-phosphat-isopropylester wird durch Polyacrylamid-Gelelektrophorese oder HPLC gereinigt.

b) Herstellung eines Oligonucleotids der Formel I b ($R^1$=$R^2$=H, Z=O-i-$C_3H_7$, a=U=V=W=X=Y=Y'=O)

d(CpGpTpCpCpApTpGpTpCpGpGpCpApApApCpApGpCpTp-O-i-$C_3H_7$)

Die Synthese erfolgt analog Beispiel 4a, jedoch mit unterschiedlichen Nucleobasen im Monomer. In den Syntheseschritten 1 bis 8 wird das Monomer im allgemeinen als 5'-O-Dimethoxytrityl-nucleosid-3'-phosphorigsäure-β-cyanoethylester-dialkylamid eingesetzt, wobei die Amino-Funktion von Adenin (Ade), Cytosin (Cyt) oder Guanin (Gua) mit geeigneten Schutzgruppen versehen sind. In diesem Beispiel verwendet man $N^6$-Benzoyl-Ade ($Ade^{Bz}$), $N^4$-Benzoyl-Cyt ($Cyt^{Bz}$) und $N^2$-Isobutyryl-Gua ($Gua^{iBu}$). Der Kettenaufbau erfolgt ausgehend vom Träger der Formel VIIa-1 (B' = Thy,
W = O, Z = O-i-$C_3H_7$) wie in Beispiel 4a beschrieben, indem die entsprechenden Monomeren entsprechend obiger Sequenz ankondensiert werden. Zur Abspaltung der Amino-Schutzgruppen erfolgt jedoch eine zusätzliche Ammoniak-Behandlung (16 Stunden bei 50°C).

c) Herstellung eines Oligonucleotids der Formel I c ($R^1$=$R^2$=H, Z=O-$CH_2CH_3$, a=U=V=W=X=Y=Y'=O)

d(CpGpTpCpCpApTpGpTpCpCpGpCpApApApCpApGpCp-O-$CH_2CH_3$)

Ausgehend vom Träger der Formel VIIa-3 (B'=$Cyt^{iBu}$, W=O, Z=O-$C_2H_5$), dessen Darstellung mit Hilfe des Monomers der Formel VIIIa nach Methode A (Beispiel 3a) erfolgt, wird die Synthese analog zu Beispiel 4b durchgeführt. Jedoch verwendet man zur Herstellung basenlabiler Substitutionen (wie hier für Z=O-$C_2H_5$) vorteilhaft die labileren Amino-Schutzgruppen $N^6$-Phenoxyacetyl-Ade ($Ade^{PAC}$), $N^4$-Isobutyryl-Cyt ($Cyt^{iBu}$), $N^2$-Phenoxyacetyl-Gua ($Gua^{PAC}$), welche am Ende der Synthese leichter zu spalten sind. Die Entfernung der Schutzgruppen mit Ammoniak dauert dann lediglich 2 Stunden bei 50°C. Behandelt man das Produkt weitere 6 Stunden bei 50°C mit Ammoniak, erhält man als Nebenprodukt durch Spaltung des Phosphatethylesters etwa 5 bis 10 Prozent des

Oligonucleotid-3'-phosphats.

d) Herstellung eines Oligonucleotids der Formel I d ($R^1$=$R^2$=H, Z=O-$(CH_2)_{17}CH_3$, a=U=V=X=Y=Y'=O; W=O, ausgenommen die beiden letzten 5'-terminalen Phosphorothioat-Internucleotid-Bindungen, worin W=S ist (gekennzeichnet als $p_S$))

$$d(Cp_SGp_STpCpCpApTpGpTpCpGpGpCpApApApCpApGpCp\text{-}O\text{-}(CH_2)_{17}CH_3)$$

Ausgehend vom Träger der Formel VIIa-4 (B'=$Cyt^{iBu}$, W=O, Z=O-$(CH_2)_{17}CH_3$), dessen Darstellung mit Hilfe des Monomers der Formel VIIId nach Methode A (Beispiel 3a) erfolgt, wird die Synthese analog wie in Beispiel 4c beschrieben mit den labileren Schutzgruppen durchgeführt. Nach der Kupplung des zweitletzten (G) und des letzten Nucleotids (C) wird anstelle von Jodwasser eine TETD-Lösung (0.4 M Tetraethylthiuramdisulfid in Acetonitril) zur Schwefeloxidation eingesetzt. Durch 2-stündige Ammoniakbehandlung werden die Schutzgruppen entfernt. Man erhält ein Oligonucleotid der Formel Id mit zwei 5'-terminalen Phosphorothioat-Internucleotid-Bindungen und einem 3'-O-n-Octadecyl-phosphatester-Rest.

e) Herstellung eines Oligonucleotids der Formel I e ($R^1$=$R^2$=H, Z=$CH_3$, a=V=W=X=Y=Y'=O; U=O, ausgenommen die beiden 5'-terminalen Methylphosphonat Internucleotid-Bindungen, worin U=$CH_3$ ist (gekennzeichnet als $P_{Me}$))

$$d(Cp_{Me}Gp_{Me}TpCpCpApTpGpTpCpGpGpCpApAPApCpApGpCpTp_{Me})$$

Ausgehend vom Träger der Formel VIIa-5 (B'=Thy, W = O, Z=$CH_3$), dessen Darstellung mit Hilfe des Monomers der Formel VIII p nach Methode A (Beispiel 3a) erfolgt, wird die Synthese analog zu Beispiel 4c durchgeführt. Zur Kupplung der letzten beiden Nucleotid-Einheiten (G und C) werden anstelle der normalen cyanoethyl-geschützten Monomeren (Formel VIII, Z=$OCH_2CH_2CN$) die entsprechenden Methylphosphonamidite (Formel VIII, Z= $CH_3$) eingesetzt. Der Spaltung vom Träger (1.5 Stunden Raumtemperatur) mit konz. Ammoniak folgt eine 6-stündige Behandlung mit Ethylendiamin/Ethanol/Wasser (5:4:1; v:v:v) zur Freisetzung der Amino-Funktionen der Basen. Es resultiert ein Oligonucleotid-3'-methylphosphonat mit zwei 5'-terminalen Methylphosphonat Internucleotid-Bindungen der Formel I e.

f) Herstellung eines Oligonucleotids der Formel I f ($R^1$=$R^2$=H, Z=$CH_3$, X=S, a=U=V=W=Y=Y'=O)

$$d(CpGpTpCpCpApTpGpTpCpGpGpCpApApApCpApGpCp_{(S)Me})$$

Ausgehend vom Träger der Formel IVa aus Beispiel 1a wird im ersten Reaktionszyklus das Methylphosphonamidit der Formel VIII (Z=$CH_3$, B'=$Cyt^{iBu}$, $R_5$=$R_6$=i-$C_3H_7$) wie in Beispiel 3a beschrieben gekuppelt. Die Oxidation wird mit TETD durchgeführt. Dann wird wie in Beispiel 3c weitersynthetisiert. Nach der Abspaltung der Schutzgruppen analog Beispiel 3e, erhält man ein Oligonucleotid-3'-methylphosphonothioat der Formel I f.

g) Herstellung eines Oligonucleotids der Formel I g ($R^1$=$R^2$=H, Z=X=S, a=U=V=W=Y=Y'=O)

$$d(CpGpTpCpCpApTpGpTpCpGpGpCpApApApCpApGpCp_{(S)S}$$

Anolog der in Beispiel 4b beschriebenen Synthese ausgehend vom Träger der Formel IVa aus Beispiel 1a, jedoch mit dem Unterschied, daß im ersten Kondensationsschritt ein Nucleosid-3'-phosphoramidit der Formel VIII (Z=2.4-Dichlorthiobenzyl; $R^5$=$R^6$=Ethyl) anstelle des Methylphosphonamidits eingesetzt wird. Die Einführung des zweiten S-Atoms gelingt wiederum durch Oxidation mit TETD (0.4M in Acetonitril). Die Spaltung der Dichlorbenzylthio-Gruppe erfolgt in bekannter Weise mit Thiophenol/Triethylamin. Nach der Abspaltung der Schutzgruppen mit konz. Ammoniak erhält man ein Oligonucleotid-3'-phosphorodithioat der Formel I g.

h) Herstellung eines Oligonucleotids der Formel I h ($R^1$=$R^2$=H, Z= p-Nitrophenylethyl-2-oxy, a=U=V=W=X=Y=Y'=O)

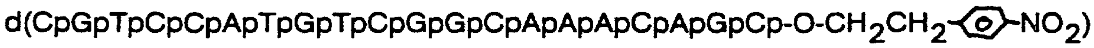

Ausgehend vom Träger der Formel VIIa-6 (B'=Cyt[Bz], W = O, Z= p-Nitrophenylethyl-2-oxy), dessen Darstellung mit Hilfe des Monomers der Formel VIIIf nach Methode A (Beispiel 3a) erfolgt, wird die Synthese analog wie in Beispiel 4b durchgeführt. Nach Abspaltung der Schutzgruppen durch 10-stündige Ammoniakbehandlung bei 55°C erhält man ein Oligonucleotid-3'-O-(p-nitrophenylethyl)phosphat der Formel I h.

i) Herstellung eines Oligonucleotids der Formel I i ($R^1$=$R^2$=H, Z=3-Pyridylpropan-3-oxy, a=U=V=W=X=Y=Y'=O)

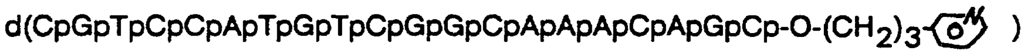

Ausgehend vom Träger der Formel VIIa-7

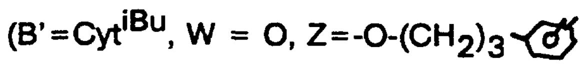

, der wie in Beispiel 3a beschrieben mit Hilfe des Amidits der Formel VIII e hergestellt wurde, erfolgt die Oligonucleotid-Synthese analog Beispiel 4c.

k) Herstellung eines Oligonucleotids der Formel I k ($R^1$=$R^2$=H, Z=-O-$(CH_2CH_2O)_3CH_3$, a=U=V=W=X=Y=Y'=O)

d(GpApGpGpApCpGpTpTpCpCpTpCpCpTpGpCpGpGpGpApApGpGpCp-O-$(CH_2CH_2O)_3CH_3$)

Ausgehend vom Träger der Formel VIIa-8 (B'=Cyt[Bz], W = O, Z=-O-$(CH_2CH_2O)_3CH_3$, der wie in Beispiel 3a beschrieben mit Hilfe des Amidits der Formel VIIIg hergestellt wurde, erfolgt die Oligonucleotid-Synthese entsprechend obiger Sequenz analog Beispiel 4b.

l) Herstellung eines Oligonucleotids der Formel II ($R^1$=$R^2$=H, Z=-O-$(CH_2CH_2O)_5(CH_2)_4CH_3$, a=U=V=W=X=Y=Y'=O)

d(CpGpTpCpCpApTpGpTpCpGpGpCpApApApCpApGpCp-O-$(CH_2CH_2O)_5(CH_2)_4CH_3$)

Ausgehend vom Träger der Formel VIIa-9 (B'=Cyt[Bz], W = O, Z= -O-$(CH_2CH_2O)_5(CH_2)_4CH_3$, der wie in Beispiel 3a beschrieben mit Hilfe des Amidits der Formel VIIIi hergestellt wurde, erfolgt die Oligonucleotid-Synthese analog Beispiel 4b.

m) Herstellung eines Oligonucleotids der Formel I m ($R^1$=$R^2$=H, Z= -O-$(CH_2CH_2O)_8(CH_2)_{13}CH_3$, a=U=V=W=X=Y=Y'=O)

d(CpGpTpCpCpApTpGpTpCpGpGpCpApApApCpApGpCp-O-$(CH_2CH_2O)_8(CH_2)_{13}CH_3$)

Ausgehend vom Träger der Formel VIIa-10 (B'=Cyt[Bz], W = O,

$Z=-O-(CH_2CH_2O)_8(CH_2)_{13}CH_3$, der wie in Beispiel 3a beschrieben mit Hilfe des Amidits der Formel VIIIk hergestellt wurde, erfolgt die Oligonucleotid-Synthese analog Beispiel 4b.

n) Herstellung eines Oligonucleotids der Formel I n ($R^1=R^2=H$, $Z=-(CH_3)N(CH_2)_2N(CH_3)_2$, a=U=V=W=X=Y=Y'=O)

d(CpGpTpCpCpApTpGpTpCpGpGpCpApApApCpApGpCp-$N(CH_3)(CH_2)_2N(CH_3)_2$

Ausgehend vom Träger der Formel IVc aus Beispiel 1c wird die Oligonucleotid-Synthese wie in Beispiel 4a beschrieben durchgeführt mit der Ausnahme, daß zur ersten Kondensationsreaktion ein Methoxy-Phosphoramidit der Formel VIII (B'=$Cyt^{i-Bu}$, $Z=OCH_3$, $R^5=R^6=N(i-C_3H_7)_2$ eingesetzt wird und die oxidative Amidierung mit einer 0.1 M Jodlösung in THF/N,N',N'-Trimethylethylendiamin (2:1; v:v) für zweimal 15 Minuten erfolgt. Der basenstabile Sulfid-Träger wird nach Aufbau der Oligonucleotidsequenz mit Hilfe von $NaJO_4$ in an sich bekannter Weise zum basenlabilen Sulfon-Träger oxidiert. Die Abspaltung vom Träger sowie der Schutzgruppen (PAC für Ade und Gua; i-Bu für Cyt) erfolgt mit t-Butylamin/ Methanol (1:1, v:v) bei 50°C für 16 Stunden. Man erhält ein Oligonucleotid-3'-trimethylethylendiamin-phosphoramidat der Formel In.

o) Herstellung eines Oligonucleotids der Formel Io ($R^1=R^2=H$, $Z=-HN(CH_2)_2O-CH_3$, a=U=V=W=X=Y=Y'=O)

d(CpGpTpCpCpApTpGpTpCpGpGpCpApApApCpApGpCp-$HN(CH_2)_2O-CH_3$)

Analog Beispiel 4n, wobei die oxidative Amidierung mit einer 0.1 M Jodlösung in THF/ 2-Methoxy-ethylamin (2:1; v:v) für zweimal 15 Minuten erfolgt. Nach der Abspaltung der Schutzgruppen erhält man ein Oligonucleotid-3'-(2-methoxyethyl)-phosphoramidat der Formel I o.

p) Herstellung eines Oligonucleotids der Formel I p ($R^1$=Formel II, $R^2$=H, Z=S, a=U=V=W=X=Y=Y'=Z'=O)

d($p_S$CpGpTpCpCpApTpGpTpCpGpGpCpApApApCpApGpCp$_S$)

Die Synthese wird ausgehend vom Träger der Formel IV a wie in Beispiel 4b beschrieben durchgeführt. Jedoch wird nach der Kupplung des ersten Bausteins (Formel VIII; B'=$Cyt^{Bz}$; $Z=O-CH_2CH_2CN$; $R^5=R^6=N(i-C_3H_7)_2$) mit TETD oxidiert. Nach der Entfernung der DMTr-Schutzgruppe, der zuletzt addierten Base wird die freie 5'-Hydroxy-Gruppe mit dem Bis-Cyanoethyloxy-phosphoramidit der Formel IX ($R^9=N(i-C_3H_7)_2$, $Z''=R^{10}=OCH_2CH_2CN$ phosphityliert und anschließend mit TETD zum Thiophosphat oxidiert. Die Cyanoethyl-Schutzgruppen werden bei der Ammoniakbehandlung eliminiert. Es resultiert ein Oligonucleotid-3'5'-bis-thiophosphat der Formel I p.

q) Herstellung eines Oligonucleotids der Formel I q ($R^1$=Formel II, $R^2$=H, $Z=O-i-C_3H_7$, a=U=V=W=X=Y=Y'=Z'=O)

d($i-C_3H_7$-O-pCpGpTpCpCpApTpGpTpCpGpGpCpApApApCpApGpCpTp-O

-$i-C_3H_7$)

Die Synthese wird wie in Beispiel 4b beschrieben durchgeführt. Nach der Entfernung der DMTr-Schutzgruppe, der zuletzt addierten Base wird die freie 5'-Hydroxy-Gruppe mit dem Cyanoethyloxy-i-propyloxy-phosphoramidit der Formel IX ($R^9=N(i-C_3H_7)_2$, $R^{10}=OCH_2CH_2CN$, $Z''=O-i-C_3H_7$ phosphityliert und anschließend mit Jodwasser oxidiert. Es resultiert ein Oligonucleotid-3'5'-bis-isopropylphosphatester der Formel I q.

r) Herstellung eines Oligonucleotids der Formel Ir ($R^1$=Formel II, $R^2$=H, $Z=n-C_8H_{17}$, a=U=V=W=X=Y=Y'=Z'=O)

d($CH_3(CH_2)_7$-pCpGpTpCpCpApTpGpTpCpGpGpCpApApApCpApGpCpTp-$(CH_2)_7$ $CH_3$)

Ausgehend vom Träger der Formel VIIa-2 (B'=Thy, W=O, Z=$(CH_2)_7CH_3$, dessen Darstellung in Beispiel 3b beschrieben ist, wird die Synthese analog Beispiel 4c durchgeführt. Nach Entfernung der DMTr-Schutzgruppe, der zuletzt addierten Base wird die freie 5'-Hydroxy-Gruppe mit n-Octyldichlorphosphan der Formel IX (Z"= $(CH_2)_7CH_3$, $R^9$=$R^{10}$=Cl) unter Verwendung von DIPEA (Diisopropylethylamin) phosphityliert. Nach Oxidation und Hydrolyse wird das Oligonucleotid wie in Beispiel 4e vom Träger abgespalten. Man erhält ein Oligonucleotid-3'5'-bis-(n-octylphosphonat) der Formel I r.

Beispiel 5: Überprüfung auf Nuclease-Stabilität

10 nmol des zu untersuchenden Oligonucleotids werden in 450 µl 20%-igem fötalem Kälberserum in RPMI-Medium und 50 ml bidestilliertem Wasser gelöst und bei 37°C inkubiert. Dann werden sofort und nach 1, 2, 4, 7 und 24 Stunden 10 µl Proben für die Gelelektrophorese bzw. 20 µl Proben für die HPLC entnommen, zum Abbruch der Reaktion mit 5 µl bzw. 10 µl Formamid versetzt und 5 Minuten auf 95°C erhitzt. Für die Gelelektrophorese werden die Proben auf ein 15% Polyacrylamid-Gel (2%BIS) aufgetragen und dieses bei etwa 3000 Voltstunden entwickelt. Die Banden werden durch die Silberfärbung sichtbar gemacht. Zur HPLC Analyse werden die Proben auf eine Gen-Pak Fax HPLC Säule (Fa. Waters/Millipore) gespritzt und bei 1 ml/min mit 5 bis 50 % Puffer A in B chromatographiert (Puffer A: 10mM Natrium-dihydrogenphosphat, 0.1M NaCl in Acetonitril/Wasser 1:4 (v:v) pH 6.8; Puffer B: wie A, jedoch 1.5 M NaCl).

Beispiel 6: Antivirale Aktivität

Die antivirale Wirkung der erfindungsgemäßen Verbindungen wird in in vitro Versuchen geprüft. Dazu werden die erfindungsgemäßen Verbindungen in verschiedenen Verdünnungen zu Zellkulturen von HeLa- und Verozellen in Mikrotiterplatten gegeben. Nach 3 h werden die Kulturen mit verschiedenen humanpathogenen Viren (z.B: Herpesviren HSV-1, HSV-2, Orthomyxoviren Influenza A2, Picornaviren Rhinovirus 2) infiziert. 48 bis 72 h nach der Infektion wird der Therapieerfolg anhand des cytopathogenen Effektes mikroskopisch und nach Neutralrot-Aufnahme (Farbtest nach Finter) photometrisch bestimmt (Finter, N.B., In "Interferones", N.B. Finter et al., North Holland Publishing Co., Amsterdam, 1966). Die minimale Konzentration, bei der etwa die Hälfte der infizierten Zellen keinen cytopathogenen Effekt zeigen, wird als minimale Hemmkonzentration (MHK) betrachtet.

Beispiel 7: Herstellung des Phosphitylierungsreagenzes DMTr-O-$CH_2CH_2$-S-$CH_2CH_2$O-P-{$OCH_2CH_2CN$}{N(i-$C_3H_7$)$_2$ (Anspruch 9; X'=O, x'=null, $R^9$=$OCH_2CH_2CN$, $R^{10}$=N(i-$C_3H_7$)$_2$

Das Bis-Hydroxyethylsulfid (3.05 gr) wird in 75 absolutem Pyridin gelöst und diese Lösung auf 0° C gekühlt. Unter Rühren werden dann während einer Stunde das Dimethoxytritylchlorid (8.04 gr) in 60 ml abs. Pyridin gelöst zugetropft. Nach Erwärmen der Reaktionsmischung auf Raumtemperatur wird diese noch 1.5 Stunden weitergerührt. Die Lösung wird mit Wasser (5 ml) versetzt und dann im Vakuum konzentriert. Den Rückstand löst man in 250 ml Methylenchlorid, extrahiert diese Lösung dreimal mit je 125 ml 0.1 M Phosphat-Puffer pH7, trocknet die organische Phase über Natriumsulfat und konzentriert sie im Vakuum. Das Rohprodukt wird mit Hilfe von Essigester/n-Heptan/Triethylamin (Gradient 6:14:1 bis 2:2:1, v:v:v) über eine Kieselgelsäule chromatographiert. Man erhält 5.3 gr des Bis-Hydroxyethylsulfid-mono-(dimethoxytrityl)-ethers DMTr-O-$CH_2CH_2$-S-$CH_2CH_2$OH (52%).

Eine Lösung dieser Dimethoxytrityl-Verbindung (1.06 gr) und Tetrazol (88 mg) in 12.5 ml absolutem Acetonitril wird langsam (20 min.) zu einer Lösung von Cyanoethoxy-di-isopropylamino-phosphan (0.75 gr) in abs. Acetonitril (20 ml) getropft. Nach weiteren 3 Stunden Reaktionszeit wird die Reaktionslösung mit 95 ml Methylenchlorid verdünnt und mit 5 %iger Natriumcarbonatlösung (65 ml) gewaschen. Die organische Phase wird über Natriumsulfat getrocknet und im Vakuum eingeengt. Den Rückstand reinigt man durch Chromatographie über eine Kieselgelsäure mit Essigester/n-Hexan/Triethylamin (11:8:1, v:v:v). Man erhält 1.25 gr (80 %) des gewünschten Phosphitylierungsreagenzes ($^{31}$P-NMR: δ 148 ppm [d], 99 % vom Gesamt-Phosphorgehalt).

## Patentansprüche

1. Oligonucleotidanalogon der Formel I

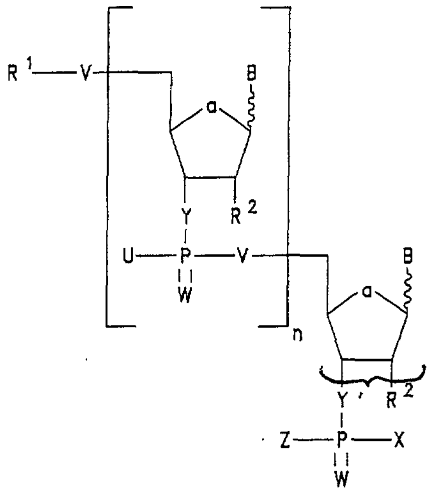

(I)

sowie dessen physiologisch verträglichen Salze, **dadurch gekennzeichnet, daß**

$R^1$ Wasserstoff, $C_1$-$C_{18}$-Alkyl, $C_2$-$C_{18}$-Alkenyl, $C_2$-$C_{18}$-Alkinyl, $C_2$-$C_{18}$-Alkylcarbonyl, $C_3$-$C_{19}$-Alkenylcarbonyl, $C_3$-$C_{19}$-Alkinylcarbonyl, $C_6$-$C_{20}$-Aryl, ($C_6$-$C_{14}$)-Aryl-($C_1$-$C_8$)-alkyl, oder

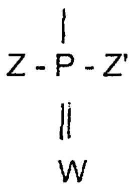

bedeutet;

$R^2$ Wasserstoff, Hydroxy, $C_1$-$C_{18}$-Alkoxy, Halogen, Azido oder $NH_2$ bedeutet;
B für eine in der Nucleotidchemie übliche Base steht;
a für Oxy oder Methylen steht;
n eine ganze Zahl von 1 bis 100 bedeutet;
W Oxo, Thioxo oder Selenoxo bedeutet;
V Oxy, Sulfandiyl oder Imino bedeutet;
Y Oxy, Sulfandiyl, Imino oder Methylen bedeutet;
Y' Oxy, Sulfandiyl, Imino, $(CH_2)_m$ oder $V(CH_2)_m$ ist, worin
m eine ganze Zahl von 1 bis 18 bedeutet;
X Hydroxy oder Mercapto bedeutet;
U Hydroxy, Mercapto, SeH, $C_1$-$C_{18}$-Alkoxy, $C_1$-$C_{18}$-Alkyl, $C_6$-$C_{20}$-Aryl, ($C_6$-$C_{14}$)-Aryl-($C_1$-$C_8$)-alkyl, $NHR^3$, $NR^3R^4$ oder $(OCH_2CH_2)_pO(CH_2)_qCH_2R^{11}$ bedeutet, worin
$R^3$ $C_1$-$C_{18}$-Alkyl, $C_6$-$C_{20}$-Aryl, ($C_6$-$C_{14}$)-Aryl-($C_1$-$C_8$)-alkyl, 2-$(CH_2)_c$-$[NH(CH_2)_c]_d$-$NR^{12}R^{12}$, worin c eine ganze Zahl von 2 bis 6 und d eine ganze Zahl von 0 bis 6 ist, und $R^{12}$ unabhängig voneinander Wasserstoff oder $C_1$-$C_6$-Alkyl oder $C_1$-$C_4$-Alkoxy-$C_1$-$C_6$-alkyl ist;
$R^4$ $C_1$-$C_{18}$-Alkyl, $C_6$-$C_{20}$-Aryl oder ($C_6$-$C_{10}$)-Aryl-($C_1$-$C_8$)-alkyl bedeutet oder im Falle von $NR^3R^4$ zusammen mit $R^3$ und dem sie tragenden Stickstoffatom einen 5-6-gliedrigen heterozyklischen Ring bedeutet, der zusätzlich ein weiteres Heteroatom aus der Reihe O, S, N enthalten kann,
p eine ganze Zahl von 1 bis 100 ist,
q eine ganze Zahl von 0 bis 22 ist,
$R^{11}$ Wasserstoff oder eine funktionelle Gruppe bedeutet;
Z = Z'
Mercapto, $C_1$-$C_{22}$-Alkoxy, $C_1$-$C_{18}$-Alkyl,
($C_6$-$C_{14}$)-Aryl($C_1$-$C_8$)-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, $C_1$-$C_4$-Alkylamino, $C_1$-$C_6$-Alkoxy,

Hydroxy, Halogen und Cyano substituiert ist, oder einen Rest der Formel III bedeutet, und
die geschweifte Klammer andeutet, daß sich $R^2$ und der benachbarte Phosphorylrest in 2'- und 3'-Stellung oder auch umgekehrt in 3'- und 2'-Stellung befinden können,
wobei jedes Nucleotid in seiner D- bzw. L-Konfiguration vorliegen kann und sich die Base B in $\alpha$- bzw. $\beta$-Stellung befinden kann, mit der Maßgabe, daß falls Z = Mercapto, $C_1$-$C_{18}$-Alkyl oder Ethoxy ist, mindestens eine der Gruppen X, Y, Y', V, W ungleich Hydroxy, Thio, Oxy bzw. Oxo ist oder $R^1$ ungleich Wasserstoff ist.

**2.** Oligonucleotidanalogon gemäß Anspruch 1, **dadurch gekennzeichnet, daß** sich die Base B in ß-Stellung befindet, die Nucleotide in der D-Konfiguration vorliegen, $R^2$ sich in 2'-Stellung befindet und a für Oxy steht.

**3.** Oligonucleotidanalogon gemäß Anspruch 1 oder 2, **dadurch gekennzeichnet, daß**

$R^1$ Wasserstoff, $C_1$-$C_6$-Alkyl, insbesondere Methyl, oder einen Rest der Formel II bedeutet;
$R^2$ Wasserstoff, Hydroxy oder $C_1$-$C_{18}$-Alkoxy, insbesondere Wasserstoff, bedeutet;
n eine ganze Zahl von 10 bis 40, insbesondere 12 bis 30, bedeutet;
m eine ganze Zahl von 1 bis 6, insbesondere 1, bedeutet;
U Hydroxy, Mercapto, $C_1$-$C_6$-Alkoxy, $C_1$-$C_6$-Alkyl, $NR^3R^4$ oder $NHR^3$, insbesondere Hydroxy oder $C_1$-$C_6$-Alkyl, bedeutet, worin
$R^3$ $C_1$-$C_8$-Alkyl, bevorzugt $C_1$-$C_4$-Alkyl, oder Methoxyethyl ist.

**4.** Oligonucleotidanalogon gemäß einem oder mehreren der Ansprüche 1, 2 oder 3, **dadurch gekennzeichnet, daß** V, Y und Y' die Bedeutung von Oxy haben.

**5.** Oligonucleotidanalogon gemäß einem oder mehreren der Ansprüche 1-3 oder 4, **dadurch gekennzeichnet, daß** W die Bedeutung von Oxo hat.

**6.** Oligonucleotidanalogon gemäß einem oder mehreren der Ansprüche 1-4 oder 5, **dadurch gekennzeichnet, daß** U die Bedeutung von Hydroxy hat.

**7.** Oligonucleotidanalogon gemäß einem oder mehreren der Ansprüche 1-4 oder 5, **dadurch gekennzeichnet, daß** $R^1$ für Wasserstoff steht.

**8.** Verfahren zur Herstellung eines Oligonucleotidanalogons der Formel I gemäß Anspruch 1, wobei

a) eine Nucleotideinheit mit 3'(2')-terminaler Phosphor(V)-Gruppierung und freier 5'-Hydroxy- oder Mercaptogruppe mit einer weiteren Nucleotideinheit mit 3'-ständiger Phosphor(III)- oder Phosphor(V)-Nucleotideinheit mit 3-ständiger Phosphor(III)-oder Phosphor(V)-Gruppierung oder deren aktivierten Derivaten umgesetzt wird, oder
b) das Oligonucleotidanalogon durch Fragmente in gleicher Weise aufgebaut wird,

in den nach (a) oder (b) erhaltenen Oligonucleotiden zum Schutz anderer Funktionen temporär eingeführte Schutzgruppen abgespaltenwerden und die so erhaltenen Oligonucleotidanaloga der Formel I gegebenenfalls in ihr physiologisch verträgliches Salz überführt werden.

**9.** Verwendung in vitro eines Oligonucleotidanalogons gemäß einem oder mehreren der Ansprüche 1-6 oder 7 als Inhibitor der Genexpression.

**10.** Verwendung in vitro eines Oligonucleotidanalogons gemäß einem oder mehreren der Ansprüche 1-6 oder 7 als Sonde zum Nachweis von Nucleinsäuren oder als Hilfsmittel in der Molekularbiologie.

**11.** Pharmazeutische Zubereitung enthaltend ein oder mehrere Oligonucleotidanaloga der Formel I gemäß einem oder mehreren der Ansprüche 1-7, gegebenenfalls zusammen mit physiologisch verträglichen Hilfs- und/oder Trägerstoffen und/oder zusammen mit anderen bekannten Wirkstoffen.

## Claims

**1.** An oligonucleotide analog of the formula I

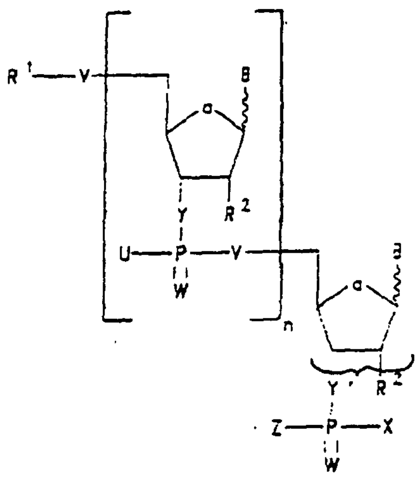

(I)

and its physiologically tolerated salts, wherein

$R^1$ is hydrogen, $C_1$-$C_{18}$-alkyl, $C_2$-$C_{18}$-alkenyl, $C_2$-$C_{18}$-alkynyl, $C_2$-$C_{18}$-alkylcarbonyl, $C_3$-$C_{19}$-alkenylcarbonyl, $C_3$-$C_{19}$-alkynylcarbonyl, $C_6$-$C_{20}$-aryl, ($C_6$-$C_{14}$)-aryl-($C_1$-$C_8$)-alkyl, or

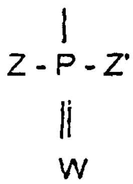

;

$R^2$ is hydrogen, hydroxyl, $C_1$-$C_{18}$-alkoxy, halogen, azido or $NH_2$;
B is a conventional base in nucleotide chemistry;
a is oxy or methylene;
n is an integer from 1 to 100;
W is oxo, thioxo or selenoxo;
V is oxy, sulfanediyl or imino;
Y is oxy, sulfanediyl, imino or methylene;
Y' is oxy, sulfanediyl, imino, $(CH_2)_m$ or $V(CH_2)_m$,
where
m is an integer from 1 to 18;
X is hydroxyl or mercapto;
U is hydroxyl, mercapto, SeH, $C_1$-$C_{18}$-alkoxy, $C_1$-$C_{18}$-alkyl, $C_6$-$C_{20}$-aryl, ($C_6$-$C_{14}$)-aryl-($C_1$-$C_8$)-alkyl, $NHR^3$, $NR^3R^4$ or $(OCH_2CH_2)_pO(CH_2)_qCH_2R^{11}$,
where
$R^3$ is $C_1$-$C_{18}$-alkyl, $C_6$-$C_{20}$-aryl, ($C_6$-$C_{14}$)-aryl-($C_1$-$C_8$)-alkyl, 2-$(CH_2)_c$-$[NH(CH_2)_c]_d$-$NR^{12}R^{12}$, where c is an integer from 2 to 6 and d is an integer from 0 to 6, and each $R^{12}$ independently of the other is hydrogen or $C_1$-$C_6$-alkyl or $C_1$-$C_4$-alkoxy-$C_1$-$C_6$-alkyl;
$R^4$ is $C_1$-$C_{18}$-alkyl, $C_6$-$C_{20}$-aryl or ($C_6$-$C_{10}$)-aryl-($C_1$-$C_8$)-alkyl, or, in the case of $NR^3R^4$, is, together with $R^3$ and the nitrogen atom carrying them, a 5-6-membered heterocyclic ring, which can additionally contain a further hetero atom selected from the group comprising 0, S, N,
p is an integer from 1 to 100,
q is an integer from 0 to 22,
$R^{11}$ is hydrogen or a functional group;

Z = Z' are mercapto, $C_1$-$C_{22}$-alkoxy, $C_1$-$C_{18}$-alkyl, ($C_6$-$C_{14}$)-aryl-($C_1$-$C_8$)-alkoxy, where aryl includes heteroaryl, and aryl is optionally substituted by 1, 2 or 3 identical or different radicals selected from the group comprising carboxyl, amino, nitro, $C_1$-$C_4$-alkylamino, $C_1$-$C_6$-alkoxy, hydroxyl, halogen and cyano, or is a radical of the formula III, and

the curved bracket indicates that $R^2$ and the neighboring phosphoryl residue can be located in the 2'-and 3'-position or else the opposite way round in the 3'- and 2'-position,
where each nucleotide can be present in its D- or L-configuration and the base B can be located in the α- or β-position, with the proviso that, if Z = mercapto, $C_1$-$C_{18}$-alkyl or ethoxy, at least one of the groups X, Y, Y', V and W is not hydroxyl, thio, oxy or oxo, or $R^1$ is not hydrogen.

**2.** An oligonucleotide analog as claimed in claim 1, wherein the base B is located in the β-position, the nucleotides are present in the D-configuration, $R^2$ is located in the 2'-position and a is oxy.

**3.** An oligonucleotide analog as claimed in claim 1 or 2, wherein

$R^1$ is hydrogen, $C_1$-$C_6$-alkyl, in particular methyl, or a radical of the formula II;
$R^2$ is hydrogen, hydroxyl or $C_1$-$C_{18}$-alkoxy, in particular hydrogen;
n is an integer from 10 to 40, in particular 12 to 30;
m is an integer from 1 to 6, in particular 1;
U is hydroxyl, mercapto, $C_1$-$C_6$-alkoxy, $C_1$-$C_6$-alkyl, $NR^3R^4$ or $NHR^3$, in particular hydroxyl or $C_1$-$C_6$-alkyl, where
$R^3$ is $C_1$-$C_8$-alkyl, preferably $C_1$-$C_4$-alkyl, or methoxyethyl.

**4.** An oligonucleotide analog as claimed in one or more of claims 1, 2 or 3, wherein V, Y and Y' have the meaning of oxy.

**5.** An oligonucleotide analog as claimed in one or more of claims 1-3 or 4, wherein W has the meaning of oxo.

**6.** An oligonucleotide analog as claimed in one or more of claims 1-4 or 5, wherein U has the meaning of hydroxyl.

**7.** An oligonucleotide analog as claimed in one or more of claims 1-4 or 5, wherein $R^1$ is hydrogen.

**8.** A process for preparing an oligonucleotide analog of the formula I as claimed in claim 1, wherein

a) a nucleotide unit having a 3'(2')-terminal phosphorus(V) grouping and a free 5'-hydroxyl or mercapto group is reacted with a further nucleotide unit having a 3' phosphorus(III) or phosphorus(V) grouping or an activated derivative thereof, or
b) the oligonucleotide analog is constructed with fragments in a similar manner,

and protective groups, which have been temporarily introduced in the oligonucleotides obtained according to (a) or (b) in order to protect other functions, are removed and the oligonucleotide analog of the formula I thus obtained is, where appropriate, converted into its physiologically tolerated salt.

**9.** The in-vitro use of an oligonucleotide analog as claimed in one or more of claims 1-6 or 7 as an inhibitor of gene expression.

**10.** The in-vitro use of an oligonucleotide analog as claimed in one or more of claims 1-6 or 7 as a probe for detecting nucleic acids or as an aid in molecular biology.

**11.** A pharmaceutical preparation containing one or more oligonucleotide analogs of the formula I as claimed in one or more of claims 1-7, where appropriate together with physiologically tolerated adjuvants and/or excipients and/or together with other known active substances.

## Revendications

**1.** Analogue oligonucléotidique de formule I

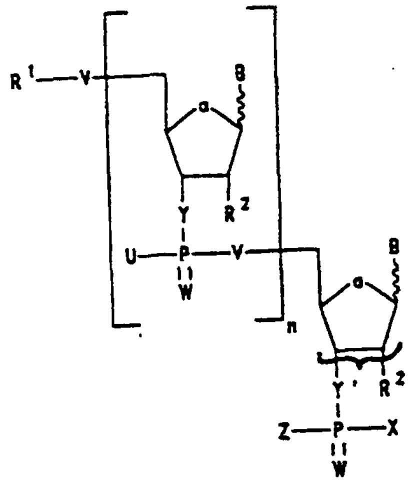

(I)

ainsi que ses sels tolérés du point de vue physiologique, **caractérisés en ce que**

$R^1$ représente un atome d'hydrogène, des groupes alkyle en $C_1$-$C_{18}$, alcényle en $C_2$-$C_{18}$, alcynyle en $C_2$-$C_{18}$, (alkyl en $C_2$-$C_{18}$)carbonyle, (alcényl en $C_3$-$C_{19}$)carbonyle, (alcynyl en $C_3$-$C_{19}$) carbonyle, aryle en $C_6$-$C_{20}$, (aryl en $C_6$-$C_{14}$)-alkyle en $C_1$-$C_8$, ou

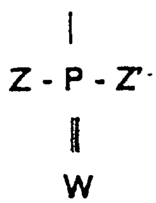

$R^2$ représente un atome d'hydrogène, des groupes hydroxy, alkoxy en $C_1$-$C_{18}$, halogène, azido ou $NH_2$ ;

B représente une base usuelle en chimie des nucléotides ;

a représente un groupe oxy ou méthylène ;

n est un entier de 1 à 100 ;

W représente des groupes oxo, thioxo ou sélénoxo ;

V représente des groupes oxy, sulfanediyle ou imino ;

Y représente des groupes oxy, sulfanediyle, imino ou méthylène ;

Y' représente des groupes oxy, sulfanediyle, imino, $(CH_2)_m$ ou $V(CH_2)_m$, où

m est un entier de 1 à 18 ;

X représente un groupe hydroxy ou mercapto ;

U représente un groupe hydroxy, mercapto, SeH, alkoxy en $C_1$-$C_{18}$, alkyle en $C_1$-$C_{18}$, aryle en $C_6$-$C_{20}$, (aryl en $C_6$-$C_{14}$)-alkyle en $C_1$-$C_8$, $NHR^3$, $NR^3H^4$ ou $(OCH_2CH_2)_pO(CH_2)_qCH_2R^{11}$, où

$R^3$ représente des groupes alkyle en $C_1$-$C_{18}$, aryle en $C_6$-$C_{20}$, (aryl en $C_6$-$C_{14}$)-alkyle en $C_1$-$C_8$, 2-$(CH_2)_c$-[NH$(CH_2)_c]_d$-$NR^{12}R^{12}$, où c est un entier de 2 à 6 et d est un entier de 0 à 6, et $R^{12}$ représentent, indépendamment l'un de l'autre, un atome d'hydrogène ou un groupe alkyle en $C_1$-$C_6$ ou (alkoxy en $C_1$-$C_4$)-alkyle en $C_1$-$C_6$ ;

$R^4$ représente des groupes alkyle en $C_1$-$C_{18}$, aryle en $C_6$-$C_{20}$ ou (aryl en $C_6$-$C_{10}$)-alkyle en $C_1$-$C_8$ ou dans le cas, où $NR^3R^4$ forme ensemble avec $R^3$ et l'atome d'azote qui les porte, un cycle hétérocylique de 5 à 6 chainons, qui peut contenir de plus un autre hétéroatome de la série des atomes d'oxygène, de soufre ou d'azote,

p est un entier de 1 à 100,

q est un entier de 0 à 22,

$R^{11}$ représente un atome d'hydrogène ou un groupe fonctionnel ;

Z = Z'

mercapto, alkoxy en $C_1$-$C_{22}$, alkyle en $C_1$-$C_{18}$, (aryl en $C_6$-$C_{14}$)-alkyle en $C_1$-$C_8$, où aryle représente aussi hétéroaryle et aryle est éventuellement substitué par 1, 2 ou 3 restes identiques ou différents pris dans le

groupe des restes carboxy, amino, nitro, (alkyl en $C_1$-$C_4$)-amino, alkoxy en $C_1$-$C_6$, hydroxy, halogène et cyano, ou un reste de formule III,
et
l'accolade indique que $R^2$ et le reste phosphoryle adjacent peuvent se trouver en positions 2' et 3' ou aussi inversément, en position 3' et 2',
où chaque nucléotide peut se présenter sous forme de sa configuration D ou L et la base B peut se trouver en position $\alpha$ ou $\beta$, à la condition que si Z = mercapto, alkyle en $C_1$-$C_{18}$ ou éthoxy, au moins un des groupes X, Y, Y', V, W ne sont pas égaux aux groupes hydroxy, thio, oxy ou oxo, ou $R^1$ n'est pas égal à un atome d'hydrogène.

**2.** Analogue oligonocléotidique selon la revendication 1, **caractérisé en ce que** la base B se trouve en position $\beta$, les nucléotides se présentent en configuration D, $R^2$ se trouve en position 2' et a représente un groupe oxy.

**3.** Analogue oligonocléotidique selon la revendication 1 ou 2, **caractérisé en ce que**

- $R^1$ représente un atome d'hydrogène, un groupe alkyle en $C_1$-$C_6$, en particulier méthyle, ou un reste de formule II ;
- $R^2$ représente un atome d'hydrogène, des groupes hydroxy ou alkoxy en $C_1$-$C_{18}$, en particulier un atome d'hydrogène ;
- n est un entier de 10 à 40, en particulier 12 à 30,
- m est un entier de 1 à 6, en particulier 1 ;
- U représente des groupes hydroxy, mercapto, alkoxy en $C_1$-$C_6$, alkyle en $C_1$-$C_6$, $NR^3R^4$ ou $NHR^3$, en particulier hydroxy ou alkyle en $C_1$-$C_6$, où
- $R^3$ représente un groupe alkyle en $C_1$-$C_8$, de préférence alkyle en $C_1$-$C_4$, ou méthoxyéthyle.

**4.** Analogue oligonocléotidique selon une ou plusieurs des revendications 1, 2 ou 3, **caractérisé en ce que** V, Y et Y' représentent un groupe oxy.

**5.** Analogue oligonocléotidique selon une ou plusieurs des revendications 1-3 ou 4, **caractérisé en ce que** W représente un groupe oxo.

**6.** Analogue oligonocléotidique selon une ou plusieurs des revendications 1-4 ou 5, **caractérisé en ce que** U représente un groupe hydroxy.

**7.** Analogue oligonocléotidique selon une ou plusieurs des revendications 1-4 ou 5, **caractérisé en ce que** $R^1$ représente un atome d'hydrogène.

**8.** Procédés pour la préparation d'un analogue oligonucléotique de formule I selon la revendication 1, où

a) on fait réagir un motif oligonucléotidique avec un groupement phosphore(V) en 3'(2') et un groupe 5'-hydroxy libre ou mercapto avec un autre motif oligonucléotidique phosphore(III) ou motif oligonucléotidique phosphore (V) en position 3' avec un groupement phosphore(III) ou phosphore(V) en position 3' ou leurs dérivés activés, ou
b) on synthétise l'analogue oligonucléotidique à l'aide de fragments de la même façon,

on sépare dans les oligonucléotides obtenus selon (a) et (b) les groupes protecteurs introduits temporairement pour la protection d'autres fonctions, et éventuellement on transforme les analogues oligonucléotidiques de formule I ainsi obtenus en leur sel toléré du point de vue physiologique.

**9.** Utilisation in vitro d'un analogue oligonucléotidique selon une ou plusieurs des revendications 1-6 ou 7 comme inhibiteur de l'expression de gène.

**10.** Utilisation in vitro d'un analogue oligonucléotidique selon une ou plusieurs des revendications 1-6 ou 7 comme sonde pour la mise en évidence d'acides nucléiques ou en tant qu'outil d'aide en biologie moléculaire.

**11.** Préparation pharmaceutique contenant un ou plusieurs analogues oligonucléotidiques de formule I selon une ou plusieurs des revendications 1 à 7, éventuellement conjointement avec des adjuvants et véhicules tolérés du point de vue physiologique et/ou autres principes actifs connus.